# EUROPÄISCHE PATENTANMELDUNG

(11) **EP 2 561 873 A1**
(43) Veröffentlichungstag der Anmeldung: **27.02.2013**
(21) Anmeldenummer: 11179040.8
(22) Anmeldetag: 26.08.2011
(51) Int. Cl.: A61K 31/437, C07D 221/00, A61K 38/00

(54) **Strukturmimetika prolinreicher Peptide und ihre Verwendung**

(71) Anmelder: Forschungsverbund Berlin e.V., 12489 Berlin (DE); UNIVERSITÄT ZU KÖLN, 50923 Köln (DE)
(72) Erfinder: Kühne, Ronald, 12683 Berlin (DE); Oschkinat, Hartmut, 13125 Berlin (DE); Schmalz, Hans-Günther, 50321 Brühl (DE); Opitz, Robert, 10969 Berlin (DE); Müller, Matthias, 10559 Berlin (DE); Reuter, Cedric, 50923 Köln (DE); Huy, Peter, 41352 Korschenbrochl (DE)
(74) Vertreter: Lange, Sven

(57) **Zusammenfassung**

Die Erfindung betrifft Verbindungen, die insbesondere als Strukturmimetika prolinreicher Peptide eingesetzt werden können und demgemäß in der Lage sind, PRM-Bindungsdomänen (proline-rich-motif binding domains) von Proteinen zu binden, die Erfindung betrifft weiterhin die Verwendung dieser Verbindungen als pharmazeutische Wirkstoffe sowie die Verwendung der pharmazeutischen Wirkstoffe zur Behandlung von bakteriellen Erkrankungen, neurodegenerativen Erkrankungen sowie Tumoren.

## Beschreibung

Die Erfindung betrifft Verbindungen, die insbesondere als Strukturmimetika prolinreicher Peptide eingesetzt werden können und demgemäß in der Lage sind, PRM-Bindungsdomänen (proline-rich-motif binding domains) von Proteinen zu binden, die Erfindung betrifft weiterhin die Verwendung dieser Verbindungen als pharmazeutische Wirkstoffe sowie die Verwendung der pharmazeutischen Wirkstoffe zur Behandlung zum Beispiel von bakteriellen Erkrankungen, neurodegenerativen Erkrankungen sowie Tumoren.

Peptide und Proteine sind essentielle Bestandteile von Organismen mit einer Vielzahl verschiedener Funktionen. Während den Proteinen vor allem biokatalytische Aufgaben (Enzyme) sowie solche als wichtige Gewebebestandteile zukommen, erfüllen die Peptide vor allem als Hormone, Neurotransmitter und Neuromodulatoren wichtige Funktionen im Organismus. Durch Bindung an membrangebundene Rezeptoren und dadurch vermittelte zellphysiologische Folgereaktionen beeinflussen Peptide die Zell-Zell-Kommunikation und kontrollieren eine Vielzahl vitaler Prozesse wie Stoffwechsel, Immunabwehr, Verdauung, Atmung, Schmerzempfinden, Reproduktion, Verhalten, Elektrolythaushalt und mehr.

Es besteht daher im Stand der Technik das Bedürfnis, die genauen Zusammenhänge im Organismus aufzuklären und gleichzeitig eine notwendige Grundlage für die Therapierbarkeit pathogener Zustände bereitzustellen. Mit einem zunehmenden Verständnis biologischer Prozesse auf molekularer Ebene hat auch die Verflechtung von Biologie und Chemie zugenommen, unterstützt durch große Fortschritte bei den analytischen Verfahren und den rechnergestützten theoretischen Methoden. All dies sind wichtige Voraussetzungen für die erfolgreiche Identifizierung von Leitstrukturen in der Wirkstoffentwicklung. Dennoch ist man vom eigentlichen Ziel, dem einfachen und effizienten *de novo*-Design von Wirkstoffen noch weit entfernt. Vielmehr bedarf es in der Regel eines großen empirischen Forschungsaufwandes, um ausgehend von natürlichen Strukturen Bibliotheken möglicher Zielsubstanzen zu synthetisieren und diese auf eine bestimmte Wirkung hin zu optimieren. Neben hohem Zeitaufwand und enormen Kosten stellt sich zudem oft heraus, dass mithilfe von Computern entwickelte Wirkstoffe in realen, sehr komplexen biologischen Systemen (z. B. Menschen) die erwünschte Wirkung nur unzureichend erzielen oder nicht zu tolerierende Nebenwirkungen haben.

Vor diesem Hintergrund bleibt die Entwicklung besonders auch peptidischer bzw. peptidmimetischer Wirkstoffe eine große Herausforderung, auch in synthetischer Hinsicht; denn im vielfältigen interdisziplinären Zusammenspiel bestimmt nicht zuletzt die organische Synthesechemie mit ihren Möglichkeiten und Beschränkungen den Zugang zu den gewünschten Zielmolekülen. Da diese in möglichst wenigen Stufen und in der Regel stereoselektiv aufgebaut werden müssen, bedarf es stetig neuer und besserer Synthesemethoden, um dieses Ziel nicht nur im Labor, sondern nachfolgend auch im Hinblick auf eine großtechnische Anwendung zu erreichen. Im Stand der Technik sind Strukturmimetika von Diprolin-Einheiten und deren Verwendung als Substituent in Prolin-reichen Peptiden mit PPII-Helix-Konformationen bekannt. Bei den bekannten Strukturen handelt es sich um solche, die ein zentrales 7-Ring-System aufweisen, das über zwei benachbarte zentrale Ring-C-Atome mit einem Seitenring verbunden vorliegt. Der Hauptvorteil des neuen Scaffolds besteht darin, dass durch die veränderte Lage der vinyliden Brücke veränderte sterische Anforderungen bei der Bindung auftreten, was insbesondere zum erheblichen Affinitätsgewinn z. B. an EVH1-Domänen oder aber bei anderen Strukturen beiträgt.

Es war daher die Aufgabe der Erfindung, Verbindungen bereitzustellen, die als Mimetika für Prolin-reiche Peptide eingesetzt werden können, insbesondere wenn diese eine PPII-HelixKonformation aufweisen. Die Prolin-Prolin-Dipeptideinheiten, insbesondere mit einer PPII-Helix-Konformation, können bevorzugt als Liganden für so genannte PRM-Bindungsdomänen fungieren (PRM = proline-rich motifs).

Das erfindungsgemäße Problem wird überraschend gelöst durch die Bereitstellung einer Verbindung gemäß der allgemeinen Formel 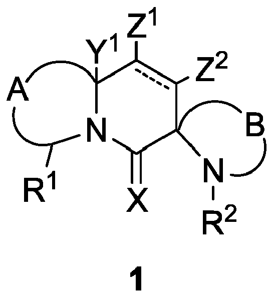 mit einem gesättigten oder ungesättigten zentralen sechsgliedrigen Ring, wobei
X = O und/oder S sind;
A, B = Ringbrücken sind;
Y¹= H, Alkyl, Fluoralkyl, Aryl und/oder Heteroaryl sind;
Z¹, Z² = H; Carbonyl; OH; O-Alkyl; O-Acyl; N-R¹R² (mit R¹ bzw. R² = H, Alkyl, Acyl, Sulfonyl); Alkyl; Acyl; Fluoralkyl; Aryl und/oder Heteroaryl sind;
R¹ = Alkyl, Acyl, Alkoxycarbonyl, Aryloxycarbonyl und/oder Aminocarbonyl (CONH₂, CONHR, CONH-Peptidyl, (mit R)) sind;
R² = H, Alkyl, Aryl, Alkylcarbonyl, Arylcarbonyl, Alkoxycarbonyl, Aminocarbonyl, Aryloxycarbonyl, Alkylsulfonyl, Arylsulfonyl, Aminoacyl und/oder Peptidyl sind.

Es war völlig überraschend, dass die erfindungsgemäßen Verbindungen die Nachteile des Standes der Technik nicht aufweisen. Neben den o. g. Nachteilen des Standes der Technik war bisher der Einsatz peptidischer Wirkstoffe als Medikamente durch eine Reihe weiterer Faktoren eingeschränkt:
1) geringe metabolische Stabilität durch Proteolyse im Magen-Darm-Trakt und im Serum,
2) geringe Zellgängigkeit
3) schlechte Resorption nach oraler Einnahme, vor allem aufgrund der hohen Molekülmasse,
4) schnelle Ausscheidung durch Leber und Nieren und
5) mangelnde Selektivität durch Wechselwirkung mit unterschiedlichen Rezeptoren.

Die erfindungsgemäßen Verbindungen können überraschend als Mimetika eingesetzt werden, die als künstlich hergestellte Stoffe in der Lage sind, insbesondere die Funktion eines Rezeptorliganden auszuüben und den biologischen Effekt eines Peptides zu imitieren (Agonist) oder zu blockieren (Antagonist). Obwohl das Prinzip der Bereitstellung von Peptidmimetika bekannt ist, sind erst sehr wenige Beispiele für PPII-Struktur-Mimetika offenbart worden, die allerdings zahlreiche Nachteile aufweisen. Es war völlig überraschend, dass Strukturmimetika mit einem zentralen 6-Ring-System bereitgestellt werden können, bei denen die Verknüpfung des Seitenrings B über ein zentrales C-Atom erfolgt, wobei diese Verbindungen mehr biologische Wirksamkeiten zeigen als die bekannten Strukturmimetika, die ein zentrales 7-Ring-System aufweisen und die Bindung des Seitenrings an zwei benachbarte zentrale Ring-C-Atome. Es war nicht vorhersehbar und auch nicht naheliegend, dass diese Änderungen dazu führen, dass die erfindungsgemäßen Strukturmimetika eingesetzt werden können, um sehr gut bioverfügbare Moleküle bereitzustellen, die eine hohe biologische Wirksamkeit aufweisen, die beispielsweise die Zellmotilität beeinflusst. Wenn die erfindungsgemäßen Moleküle in Peptide eingebaut werden, sind diese überraschend besser zellgängig als bei der Verwendung von bekannten Strukturmimetika. Die neuen Strukturmimetika sind gegenüber den bekannten Strukturmimetika z,B. erheblich besser dazu geeignet, an natürliche Bindungspartner wie die EVH1-Domäne zu binden. Es handelt sich daher bei der Erfindung um völlig neue und auch erfinderische Strukturmimetika, insbesondere der PolyprolinII-Helix. Die bekannten und die neuen Strukturmimetika unterscheiden sich unter anderem in der veränderten Position der Vinyliden-Gruppe bei verbesserten PPII-Helix mimetischen Eigenschaften. Das führt dazu, dass bei Liganden, bei denen durch die Lage der Vinyliden-Brücke eine sterische Hinderung der Bindung eintritt deutliche Verbesserungen der Affinität erreicht werden, z.B. Bindung an VASP-EVH1
- Ac-SFE-[2-Cl-F]-X-X-TEDEL-NH2 Kd =0.86 uM
- Ac-SFE-[2-Cl-F]-Spir-X-TEDEL-NH2 Kd= 0.28 uM.

Auch wenn es verschiedene Ansätze gab, das Strukturmotiv der PPII-Helix ganz oder teilweise durch synthetische Analoga zu ersetzen, war es mit den bekannten Strukturen nicht oder nur sehr bedingt möglich, ihre Wechselwirkung mit verschiedenen Proteindomänen zu untersuchen. Der Stand der Technik lehrt einen Fachmann mit durchschnittlichem Wissen auch, dass die Wechselwirkung prolinreicher Helicis mit verschiedenen Proteindomänen keine besondere biologische Bedeutung aufweist. Es ist das Verdienst der Erfinder gezeigt zu haben, dass diese Wechselwirkung bzw. ihre Modifikation mit zahlreichen Erkrankungen wie bakteriellen oder viralen Infektionen, neurodegenerativen Erkrankungen oder der Ausbildung von Tumoren einhergeht. Zahlreiche im Stand der Technik bekannte Mimetika können nur mithilfe von Katalysatoren bereitgestellt werden, die aber oft nur mit Schwierigkeiten oder gar nicht aus dem System entfernt werden können. Weiterhin sei darauf hingewiesen, dass die bekannten Produkte keine hohe Lagerbeständigkeit aufweisen und oft mit Produkten, die für die Synthese notwendig sind, kontaminiert vorliegen, was insbesondere einen Einsatz in der Medizin nicht ermöglichen oder erschweren würde. Des weiteren ist die Bindungsaffinität der bisher bekannten Mimetika nicht dergestalt, dass alle erfindungsgemäßen Aufgaben gelöst werden können.

Die Wechselwirkung von Peptidliganden mit Proteinrezeptoren spielt eine wichtige Rolle bei der Regulation biologischer Prozesse und hängt in entscheidender Weise von der Peptidgeometrie ab. Unter physiologischen Bedingungen steht die Konformation eines linearen Peptids durch Rotation um einzelne Bindungen in einem dynamischen Gleichgewicht, das vom pH-Wert und von der Temperatur abhängt. Dies führt dazu, dass die biologische Reaktivkonformation nur zu einem geringen Prozentsatz vorliegt.

Die Konformation des Peptidrückgrats wird üblicherweise durch die drei Winkel ϕ (phi), ψ (psi) und ω (omega) beschrieben. Aufgrund des partiellen Doppelbindungscharakters ist die Peptidbindung in ihrer Rotation gehindert und weist eine planare Geometrie auf, was zu zwei Vorzugskonformationen führt: der *trans-* und der *cis*- Peptidbindung mit ω = 180° bzw. ω= 0°, wobei die *trans*-Konformation energetisch günstiger ist und deshalb überwiegt.

Daher genügen zur Konformationsbeschreibung des Peptidrückgrats in erster Näherung die Torsionswinkel ϕ und ψ der Aminosäurereste. Der Winkel ϕ, der die Rotation entlang der N-C_{α}-Bindung beschreibt, wird durch die vier Atome C(=O)-N-C_{α}-C(=O) definiert. In gleicher Weise definieren N-C_{α}-C(=O)-N den Winkel ψ, der die Rotation um die C_{α}-C(=O)-Bindung beschreibt. Obwohl theoretisch eine große Anzahl verschiedener Kombinationen aus ϕ und ψ möglich ist, existieren im allgemeinen in Peptiden bestimmte Vorzugskonformationen in Abhängigkeit von Größe, Polarität und Ladung der Seitenketten, was zur Ausbildung der bekannten Sekundärstrukturen wie α-Helix, β-Faltblatt, β-Turn etc. führt.

### Die Aminosäure Prolin als Bestandteil von Peptiden

Unter den zwanzig natürlich vorkommenden Aminosäuren nimmt Prolin als einzige sekundäre Aminosäure eine Sonderstellung ein. Durch die Cyclisierung der α-Seitenkette an den Amidstickstoff ist der Torsionswinkel ϕ = (-65 ± 15°) als Bestandteil des fünfgliedrigen Ringes relativ eingeschränkt, das Peptid hat folglich weniger Rotationsfreiheitsgrade. Die zweifache Alkylierung des Stickstoffs hat einerseits zur Folge, dass das sonst übliche Amidproton (im Peptidrückgrat) fehlt und Prolin deshalb als Wasserstoffbrücken-Donor ausscheidet, andererseits ist die Carbonylgruppe besonders elektronenreich und deshalb ein besserer Wasserstoffbrücken-Akzeptor als bei anderen Aminosäuren. Durch diese geometrischen und elektronischen Eigenschaften kann Prolin keine α-Helix stabilisieren ("α-Helix-Brecher") und bildet auch keine β-Faltblattstruktur ("β-Faltblatt-Brecher") sondern ist bevorzugt in anderen typischen Sekundärstrukturen anzutreffen: den sogenannten β-Turns und der Poly-Prolin-Helix (PPII-Helix).

### Prolinreiche Motive und die PPII-Helix als Sekundärstruktur

Prolinreiche Aminosäuresequenzen findet man häufig in Peptiden, die in intrazelluläre Signaltransduktionsvorgänge involviert sind. Dabei handelt es sich um Sequenzen, in denen ausschließlich oder überwiegend Prolin auftritt (oft vier und mehr Prolineinheiten in Folge).

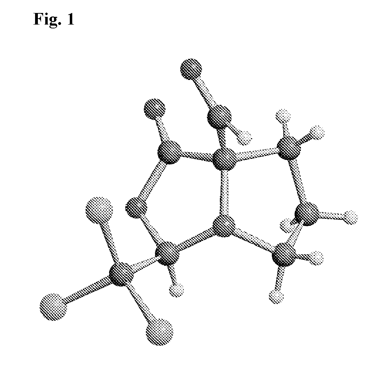

Dadurch wird die charakteristische Sekundärstruktur, die Polyprolin-Helix oder kurz PPII-Helix induziert, worunter man eine gestreckte linksgängige Helix mit den Torsionswinkeln ϕ = -78° und ψ = +146° des Peptidrückgrats versteht. Als Konsequenz ergibt sich eine pseudo-C₃-Rotationssymmetrie um die Helixachse mit genau drei Prolinresten pro Drehung im Querschnitt (Figur 1), weshalb in prolinreichen Sequenzen die Prolinreste sich bevorzugt mindestens mit einer Periodizität von drei wiederholen (z.B. PxxPxxP oder PPxPPxPPx).

Auf diese Weise sind die Prolin-Seitenketten und die Carbonylgruppen des Peptidrückgrats in regelmäßigen Intervallen dem Lösungsmittel ausgesetzt. Bedingt durch das Fehlen intramolekularer Wasserstoffbrücken sind die Carbonylgruppen besonders geeignet, intermolekulare Wasserstoffbrückenbindungen zu Rezeptorproteinen einzugehen.

Das Strukturmotiv der PPII-Helix kann aber auch dann induziert werden, wenn nicht ausschließlich Prolin anwesend ist. Besonders häufig tritt in PPII-Helices und in deren Nähe die Aminosäure Glu auf, aber auch Gln, Arg, Ala, Leu, Ser, Asp und His werden gefunden. Der bevorzugte Bindungsmodus zwischen Domäne und Ligand bestimmt, welche Prolin-Positionen streng konserviert sein müssen und welche ggf. durch andere Aminosäuren ersetzt sein können.

In einer bevorzugten Ausführungsform der Erfindung ist diese so ausgewählt, dass die durch A und B repräsentierten Brücken aus 2-4 Ringatomen bestehen, die aus der Gruppe umfassend C-, O-, S- und/oder N-Atome ausgewählt sind. Auf diese Weise ergeben sich 4, 5, 6 oder 7-gliedrige Ringe, die neben CH2-Einheiten auch -O-, -S- und -N-R mit R = H, Alkyl, oder Acyl enthalten können.

In einer weiteren bevorzugten Ausführungsform der Erfindung besitzt die Verbindung die allgemeine Formel 2 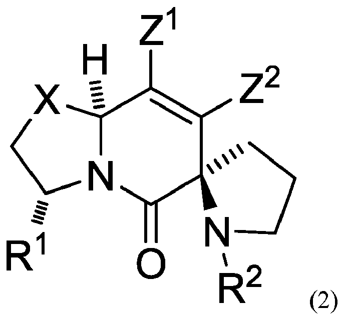 mit Z¹, Z² wie für Struktur 1 angegeben und der im Formelbild 2 gezeigten Konfiguration,
mit X = -CH₂-, -O-, -S- und/oder -NH-R.
mit R¹, R² = Alkyl, Acyl, Hetaryl und/oder Sulfonyl,

In einer weiteren bevorzugten Ausführungsform der Erfindung entspricht die Verbindung der Formel 3 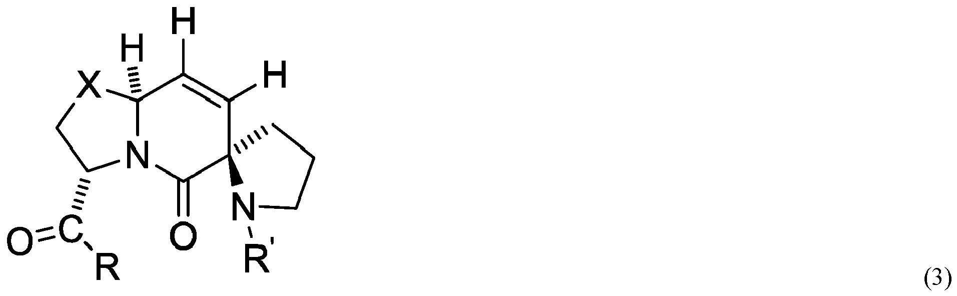 mit X = --CH₂-, -O- und/oder -S-,
mit R = NH-R", -O-R", mit R" = Peptidyl, substituierte Alkyle und/oder Hetaryl,
mit R' = Acyl, Peptidyl und/oder Sulfonyl.

Besonders bevorzugt ist in der Formel 3 R' ein Peptidyl.

Die Erfindung betrifft in einem bevorzugten Aspekt die Verwendung der genannten Verbindungen als pharmazeutische Wirkstoffe. Die Verwendung als pharmazeutischer Wirkstoff betrifft die Verwendung für chirurgische, therapeutische oder diagnostische Verfahren.

Die Erfindung betrifft in einem weiteren Aspekt ein pharmazeutisches Mittel, das die erfindungsgemäßen Verbindungen gegebenenfalls zusammen mit einem pharmazeutisch verträglichen Träger umfasst.

Bevorzugte pharmazeutische Träger sind beispielsweise Füllmittel, Streckmittel, Bindemittel, Feuchthaltemittel, Lösungsverzögerer, Sprengmittel, Resorptionsbeschleuniger, Netzmittel, Absorptionsmittel und/oder Gleitmittel.

In einem weiteren Aspekt betrifft die Erfindung die Verwendung der erfindungsgemäßen Verbindungen als Ligand für eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin.

Demgemäß betrifft die Erfindung auch die Verwendung der neuen Strukturmimetika der PolyprolinII-Helix als überraschend gute Inhibitoren von Protein-Protein-Wechselwirkungen, bei denen insbesondere SH3-Domänen, EVHI-Domänen, WW-Domänen, GYF-Domänen, UEV-Domänen und Profilin beteiligt sind. Proteine, die diese Domänen enthalten, wie z. B. VASP (EVH1-Domäne) oder YAP (WW) spielen u. a. bei der Regulation der Zellmotilität (insbesondere VASP) und der Zellproliferation (insbesondere YAP) eine bedeutsame Rolle. So ist beispielsweise VASP in hochinvasiven Brustkrebszellen stark überexprimiert. Die WW-Domänen vermittelte Wechselwirkung von YAP mit dem C-terminalen Fragment von ErbB4 führt beispielsweise zur Kernlokalisation von YAP und ist die Voraussetzung für die Funktion von YAP als Koaktivator der Zellproliferation. Demgemäß ergeben sich zahlreiche neue und überraschende Anwendungsmöglichkeiten für die erfindungsgemäßen neuen Strukturmimetika.

Bevorzugt interagieren alle diese Domänen inbesondere mit prolinreichen Sequenzen mit Affinitäten zwischen 1 und 500 µM und benötigen in bestimmten Ausführungsformen der Erfindung gegebenenfalls weitere flankierende Epitope, um die nötige Spezifität zu erreichen. Die Bindungen zwischen Liganden, Peptid und Domäne kommt bevorzugt vor allem durch die Interaktion zweier vorgeformter hydrophober Oberflächen zustande. An der Oberfläche der Domänenproteine kommt es vorteilhafterweise zu einer Ansammlung aromatischer Aminosäuren, deren Reste eine hydrophobe Bindungstasche bilden. Der prolinreiche Peptidligand weist durch die starre Natur der PPII-Helix eine geometrisch fixierte, komplementäre Struktur auf, die mit der Domänenoberfläche in Kontakt tritt. Dabei kommt es vorteilhafterweise nicht über die ganze Länge des Kernmotivs gleichermaßen zu hydrophoben Kontakten, vielmehr bildet das Ligandenpeptid eine schirmartige Struktur, die die Domäne überspannt. Einige der Prolin-Reste werden in den hydrophoben Bindungstaschen aufgenommen und interagieren so mit den aromatischen Resten der Domäne. Wenn diese Kontakte für eine biologisch relevante Bindungsstärke nicht ausreichen, werden mit Vorteil zusätzlich stabilisierende WasserstoffBrückenbindungen ausgebildet, was durch die elektronenreiche Carbonylgruppe des Prolins besonders gut ermöglicht wird.

Energetisch wird die intermolekulare Bindung durch die eingeschränkte Flexibilität der PPII-Helix begünstigt, da durch den relativ hohen Ordnungsgrad die Entropieabnahme bei der Bindungsbildung geringer ist als bei einem gewöhnlichen linearen Peptid. Zur Quantifizierung dieses Energiebeitrages kann man ein Dipeptid xP betrachten, das nur zwei der sonst üblichen vier Rotationsfreiheitsgrade um das Peptidrückgrat besitzt. Da jeder Rotationsfreiheitsgrad bei 300 K etwa 3,5 kJ/mol entspricht, ergibt sich pro xP-Dipeptid ein Energievorteil von ca. 7 kJ/mol bei der Komplexbildung.

Eine weitere Affinitätssteigerung beobachtet man auch durch die mehrfache Wiederholung der prolinreichen Sequenzen in einem einzigen Peptid, wie z.B. dem bakteriellen Oberflächenprotein ActA, das an die EVH1-Domäne (Ena-VASP-Homologie-1-Domäne) bindet.

Die EVH1-Domäne besteht aus etwa 115 Aminosäuren und kommt in einer Vielzahl signalgebender Multidomänenproteine vor. Neben einigen weiteren gehört dazu auch die Familie der Ena/VASP-Proteine, die als molekulare Adaptoren wirken und die Actindynamik des Cytoskeletts modulieren. Man unterteilt die EVH1-Domänen nach ihrer Ligandenpräferenz in drei Klassen. Die erste Klasse erkennt insbesondere spezifisch ein FPPPP-Kernmotiv, das sich in allen fokalen Adhäsionsproteinen wie zum Beispiel Vinculin und Zyxin wie auch im ActA-Protein des intrazellulären Bakteriums *Listeria monocytogenes* wiederfindet.

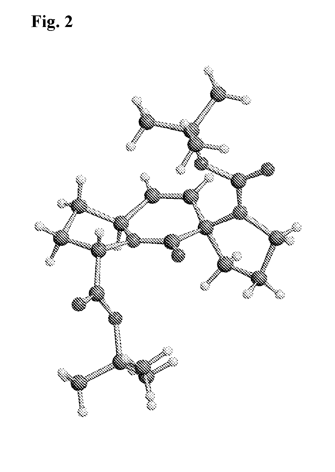

Zur Erforschung der Vorgänge auf molekularer Ebene und der resultierenden biologischen Funktionsweise wurde die dreidimensionale Domänenstruktur aufgeklärt, aber auch die Wechselwirkung mit verschiedenen Ligandenpeptiden untersucht. Dabei zeigte sich, dass die EVH1-Domänen ein Konsens-Kernmotiv FPxϕP erkennen, bei dem Phenylalanin (F) und die beiden äußeren Prolinpositionen (P) essentiell für eine Bindungsbildung sind, die beiden inneren Positionen aber durchaus Variationen zulassen (x = beliebige Aminosäuren, ϕ = hydrophobe Aminosäure). Dies ist in Figur 2 zu sehen, in der die Klasse-I-EVH1-Domäne des menschlichen VASP-Proteins mit einem kurzen Ausschnitt aus dem ActA-Peptid (₃₃₂SFEFPPPPTEDEL₃₄₄) zusammen untersucht wurde. Das zentrale FPPPP-Motiv und ein affinitätssteigerndes EL-Epitop sind umrandet und zeigen eine deutliche Beeinflussung der Bindungsstärke bei Substitution der einzelnen Positionen durch andere natürliche Aminosäuren (dunkel = gute Bindung, hell = keine Bindung).

Dass die Positionen P₀ und P₁ durch andere Aminosäuren ersetzt werden können, wobei besonders P₀ völlig unspezifisch ist, lässt sich durch die Betrachtung des Ligandenpeptids im Bindungsmodus erklären: während P₋₁ und P₂ in einer hydrophoben Bindungstasche der Domäne aufgenommen werden, befinden sich die mittleren beiden Proline in einer schirmartigen Position *über* der Domäne und haben fast keinen Kontakt mit der Domänenoberfläche. Die Carbonylgruppe von P₀ bildet allerdings eine essentielle Wasserstoffbrücke zum NH des Tryptophan-Restes W23 der EVH1-Domäne.

Weiterhin konnte anhand einer Reihe verschiedener Test-Ligandenpeptide ein Überblick über die Bindungsaffinitäten gewonnen werden. Die höchste beobachtete Bindungsaffinität (K_{D} = 45 µM) liefert der Ligand ₃₃₂SFEFPPPPTEDEL₃₄₄, (dritter der vier prolinreichen Repeats des ActA-Proteins). Bei einer Verkürzung des Liganden auf das Kernmotiv FPPPPT hingegen ließ sich keine messbare Affinität zur VASP-EVH1-Domäne mehr feststellen, eine Bindung zur Mena-EVH1-Domäne dagegen war sehr schwach, aber noch nachweisbar (417 µM).

In einer bevorzugten Ausführungsform der Erfindung werden die Verbindungen als Poly-Prolin-Mimetika eingesetzt. Vorteilhafterweise findet man prolinreiche Aminosäurensequenzen insbesondere in Peptiden, die in Signaltransduktionsvorgängen, insbesonder intrazellulären Signaltransduktionsvorgängen beteiligt sind. Im Sinne der Erfindung kann der Begriff der Mimetika auch als Analoga verstanden werden. Bevorzugt ist die Verwendung der erfindungsgemäßen Verbindungen zur Behandlung von Krankheiten, die mit einer Modifikation von intrazellulären Signaltransduktionsvorgängen assoziiert sind, die durch Poly-Prolin-Helix-Strukturen vermittelt werden, ausgewählt aus der Gruppe umfassend bakterielle Infektionskrankheiten, neurodegenerative Erkrankungen und/oder Tumorerkrankungen.

Bevorzugt handelt es sich bei den bakteriellen Erkrankungen um Erkrankungen, die durch folgende Bakterien assoziiert, insbesondere vermittelt werden: Legionellen, Streptokokken, Staphylokokken, Klebsiellen, Haemophilis influenzae, Rickettsien (Fleckfieber), Mykobakterien, Mykoplasmen, Ureaplasmen, Neisserien (Meningitis, Waterhouse-Friedrichsen-Syndrom, Gonorrhoe), Pseudomonaden, Bordetellen (Pertussis), Corynobakterien (Diphtherie), Chlamydien, Campylobacter (Durchfall), Escherichia coli, Proteus, Salmonellen, Shigellen, Yersinien, Vibrionen, Enterokokken, Clostridien, Borrelien, Treponema pallidum, Brucellen, Francisellen und/oder Leptospira, insbesondere Listerien.

Besonders bevorzugte Erkrankungen sind die, die durch Listerien ausgewählt aus der Gruppe umfassend L. monocytogenes Svl/2a, L. monocytogenes Sv4b F2365, L. monocytogenes Sv4b H7858, 178 contigs, L. monocytogenes Svl/2a F6854, 133 contigs, L. monocytogenes Sv4b, L. monocytogenes Sv4a, L. innocua Sv6a, L. welshimeri Sv6b, L. seeligeri Svl/2b und/oder L. ivanovii Sv5 ausgelöst werden oder auf die genannten bevorzugten Listerien im wesentlichen zurückgehen.

Die bevorzugten neurodegenerativen Erkrankungen sind ausgewählt aus der Gruppe umfassend Morbus Alzheimer, Morbus Parkinson, Morbus Huntington und/oder amyotrophische Lateralsklerose (ALS).

Die bevorzugten Tumorerkrankungen sind ausgewählt aus der Gruppe umfassend Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppressionbezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs; das Melanom; das Hepatom; das Neuroblastom; das Papillom; das Apudo;, das Choristom; das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in-situ-Karzinom, Krebs-2-Karzinom, Merkel-Zellen-Karzinom, Schleimkrebs, nichtkleinzelliges Bronchialkarzinom, Haferzellen-Karzinom, papilläres Karzinom, szirrhöses Karzinom, bronchiolo-alveoläres Karzinom, Bronchial-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor; Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom; Chordom; Craniopharyngiom; Dysgerminom; Hamartom; Mesenchymom; Mesonephrom; Myosarkom; Ameloblastom; Cementom; Odontom; Teratom; Thymom; Chorio-blastom; Adenokarzinom; Adenom; Cholangiom; Cholesteatom; Cylindrom; Cystadeno-carcinom; Cystadenom; Granulosa-zelltumor; Gynadroblastom; Hidradenom; Inselzelltumor; Leydig-Zelltumor; Papillom; Sertoli-Zell-Tumor; Thekazell-tumor; Leiomyom; Leiomyosarkom; Myoblastom; Myom; Myosarkom; Rhabdomyom; Rhabdomyosarkom; Ependynom; Ganglioneurom; Gliom; Medulloblastom; Meningiom; Neurilemmom; Neuroblastom; Neuroepitheliom; Neurofibrom; Neurom; Paragangliom; nicht-chromaffines Paragangliom; Angiokeratom; angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangio-myom, Lymphangiosarkom; Pinealom; Cystosarkom phyllodes; Hemangiosarkom; Lymphangiosarkom; Myxosarkom; Ovarialkarzinom; Sarkom (zum Beispiel Ewing-Sarkom, experi-mentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des ürogenitaltrakts); Neuro-fibromatose und zervikale Plattenepitheldysplasie.

In einer weiter bevorzugten Ausführungsform ist die Krebserkrankung oder der Tumor ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungen-metastasen, Lebermetastasen, Knochenmetastasen, pleurale und perikardiale Metastasen und maligne Aszites.

In einer weiter bevorzugten Ausführungsform ist die Krebserkrankung oder der Tumor ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarm-krebs, der Ovarialkarzinome, der Zervikalkarzinome, Lungen-krebs, Prostatakrebs, Nierenzellkarzinome und/oder Lebermetastasen.

Bei der Behandlung der genannten Krankheiten ist es besonders bevorzugt, das pharmazeutische Mittel, welches die erfindungsgemäßen Verbindungen umfasst, als Gel, Puder, Pulver, Tablette, Retard-Tablette, Premix, Emulsion, Aufgussformulierung, Tropfen, Konzentrat, Granulat, Sirup, Pellet, Boli, Kapsel, Aerosol, Spray und/oder Inhalat zuzubereiten und/oder anzuwenden.

Bevorzugt liegt das pharmazeutische Mittel, welches die erfindungsgemäßen Verbindungen umfasst, in einer Konzentration von 0,1 bis 99,5, bevorzugt von 0,5 bis 95,0, besonders bevorzugt von 20,0 bis 80,0 Gew.-% in einer Zubereitung vor.

Bevorzugt wird diese Zubereitung oral, subkutan, intravenös, intramuskulär, intraperitoneal und/oder topisch gesetzt.

Bevorzugt wird das pharmazeutische Mittel, das die erfindungsgemäßen Verbindungen enthält, in Gesamtmengen von 0,05 bis 500 mg pro kg, bevorzugt von 5 bis 100 mg pro kg Körpergewicht, je 24 Stunden eingesetzt.

Bevorzugt erfolgt das In-Kontakt-Bringen oral, über Injektion, topisch, vaginal, rektal und/oder nasal.

Die Erfindung betrifft auch einen Kit, der mindestens eine der erfindungsgemäßen Verbindungen und/oder eines der erfindungsgemäßen pharmazeutischen Mittel, gegebenenfalls mit einer Information zum Kombinieren der Inhalte des Kits, umfasst - z. B. einen Beipackzettel oder eine Internet-Adresse, die auf Homepages mit weiteren Informationen verweist etc. Die Information zur Handhabung des Kits kann beispielsweise ein Therapie-Schema für die o. g. Krankheiten, insbesondere für die bevorzugten Krankheiten umfassen. Die Information kann jedoch auch Angaben darüber umfassen, wie die erfindungsgemäßen Erzeugnisse innerhalb einer Diagnose der genannten Krankheiten einzusetzen sind. Der erfindungsgemäße Kit kann auch in der Grundlagenforschung verwendet werden.

Die Erfindung betrifft demgemäß auch die Verwendung des Kits zur Prophylaxe und/oder Therapie von neurodegenerativen Erkrankungen, bakteriellen Infektionserkrankungen oder Tumorerkrankungen.

### Figuren

**Fig. 1****:** Kristallstruktur des Aldehyds **104.**
**Fig. 2****:** Kristallstruktur des Bicyclus **117.**

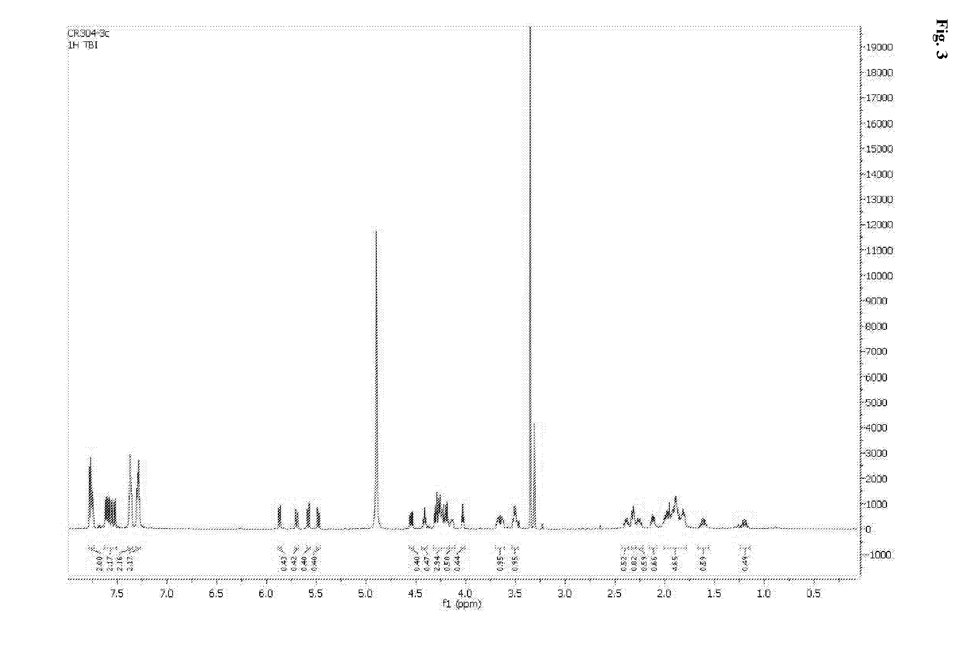

**Fig. 3****:** ¹H-NMR-Spektrum des Diprolin-Bausteins **85** (600 MHz, MeOH-d⁴, RT).

Im Folgenden soll die Erfindung anhand der Synthese des Diprolin-Mimetikums **85** näher erläutert werden, ohne auf dieses beschränkt zu sein.

Motivation war die Suche nach Molekülen, die in der Lage sein sollten, mit hoher Affinität an die EVH1-Domäne zu binden und so die nativen prolinreichen Sequenzen der Ligandenpeptide als Bindungspartner zu ersetzen. Auf Basis von Molecular-Modeling-Studien zur Interaktion von Ligandenpeptiden mit der VASP-EVH1-Domäne wurde Verbindung **85** entworfen, die als vielversprechendes Modul (Dipeptidmimetikum) in Testpeptide eingebaut werden könnte, um dort zwei benachbarte Prolin-Positionen des FPPPP-Kernmotivs zu ersetzen. Leitlinien des Struktur-Designs waren (1) eine geometrisch fixierte, helicale Struktur (Tricyclus), (2) eine größtmögliche Übereinstimmung der Bindungswinkel und -abstände im Vergleich zu denen des natürlichen Prolin-Prolin-Dipeptids (in der PPII-HelixKonformation), (3) eine zentrale Wasserstoffbrücken-Akzeptor-Funktion in Form einer Carbonylgruppe, und (4) eine Aminosäure-artige Gesamtstruktur mit einem Fmoc-geschütztem N-Terminus und einem freiem C-Terminus, um den geplanten Einbau in Peptide methodisch einfach zu gestalten. Diese Anforderungen werden von dem nachfolgend abgebildeten Molekül **85** erfüllt, das ein konformationell eingeschränktes Analogon zweier konsekutiver Proline in der PPII-Helix darstellt. Durch den Einbau der Z-konfigurierten Olefinbrücke werden die Prolinringe in der vermuteten biologisch aktiven Konformation stabilisiert, der zentrale Sechsring gewährleistet eine perfekte Fixierung der trans-Amidbindung.

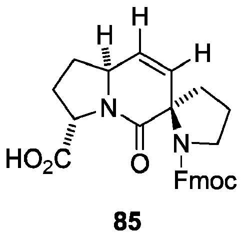

Zur Bereitstellung dieser Verbindung in substanziellen Mengen musste ein gangbarer, stereoselektiver Syntheseweg ausgearbeitet werden. Hierfür wurde eine Strategie gewählt, bei der das Zielmolekül in konvergenter Weise (retrosynthetische Zerlegung des zentralen Sechsrings, s. Schema 20) auf zwei Vinylprolinderivate vom Typ **100** und **101** zurückgeführt wird. Diese könnten zunächst durch eine Peptidkupplung miteinander verknüpft werden, bevor dann der Tricyclus durch Olefin-Metathese geschlossen würde. Ringschlussmetathesen zu Sechsringen sind in der Literatur hinreichend bekannt. Die beiden unterschiedlich substituierten Vinylprolin-Derivate **100** und **101** wiederum müssten jeweils stereoselektiv synthetisiert werden. Als Startmaterial könnte in beiden Fällen L-Prolin dienen.

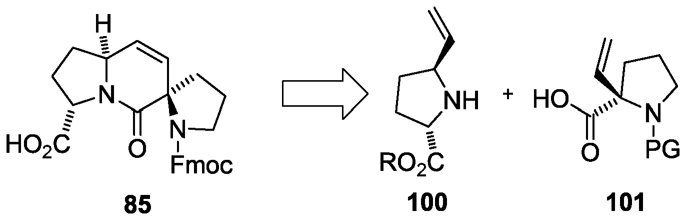

### Schema 20

Als Baustein vom Typ **101** wurde das spiro-2-Vinylprolin **107** mit einer N-Boc-Schutzgruppe avisiert. Dessen Synthese war bereits 2006 durch Gmeiner und Bittermann publiziert worden (J. Org. Chem., 2006, 71, 97-102.). (Schema 31).

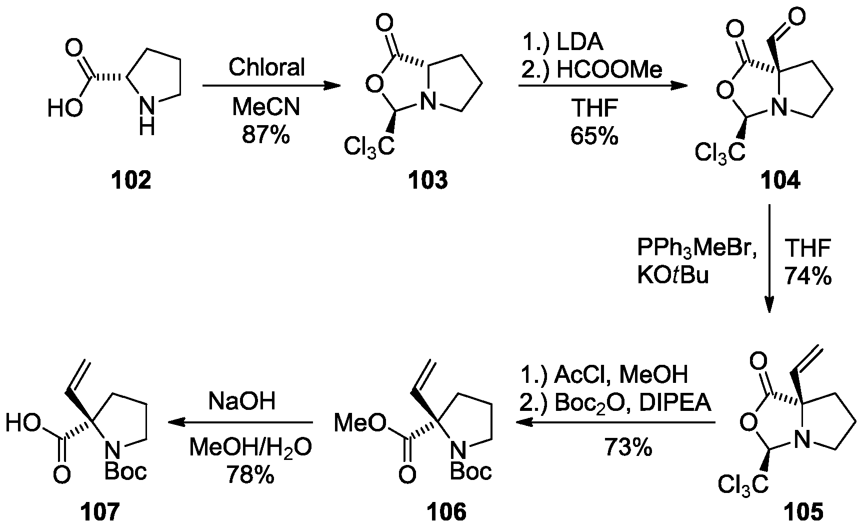

### Schema 31: Synthese des Bausteins 107 durch Gmeiner und Bittermann (J. Org. Chem., 2006, 71, 97-102.).

Diese im Jahre 2006 von Seiten Dritter publizierte Synthese (J. Org. Chem., 2006, 71, 97-102.) wurde größtenteils unverändert übernommen. Die publizierte Vorschrift konnte zunächst ohne größere Probleme im Multigrammmaßstab durchgeführt werden (Schema 41).

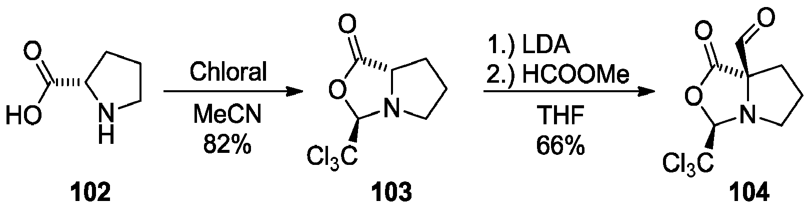

### Schema 41: Synthese des Aldehyds 104

Schützung des *L*-Prolins unter Verwendung von Chloral gab das gewünschte Produkt **103** als ein Diastereomer in 82% Ausbeute. Die folgende Formylierung konnte ebenfalls mit einer guten Ausbeute von 66% durchgeführt werden. Um den Aldehyd **104** als reines Diastereomer zu erhalten, war vor allem die äußerst langsame Zugabe des Ameisensäuremethylesters mittels Dosierpumpe notwendig. Die korrekte Konstitution und die absolute Konfiguration des Aldehyds **104** wurde durch eine Röntgen-Kristallstrukturanalyse bestätigt

Die nachfolgende Wittig-Reaktion nach dem publizierten Protokoll lieferte das gewünschte Produkt **105** reproduzierbar, in einer Ausbeute von 38% (Schema 42).

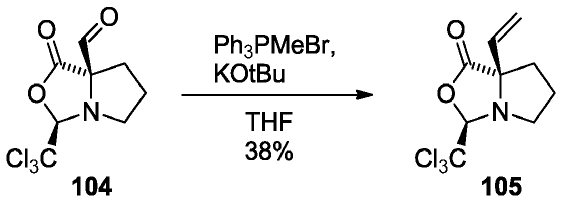

### Schema 42: Synthese von 105 durch Wittig-Reaktion.

Durch Verwendung der Nysted-Methylenierung konnte das gewünschte Olefin **105** in 67% Ausbeute erhalten werden (Schema 43)

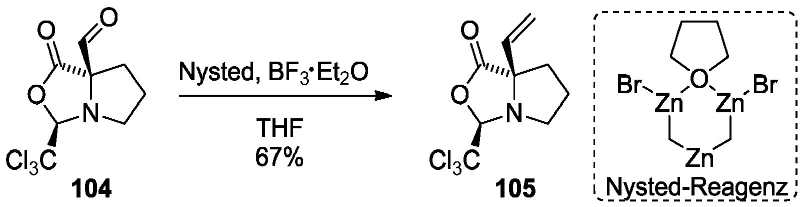

### Schema 43: Nysted-Methylenierung zum Olefin 105.

Die anschließende Umschützung zum Methylester **106** erfolgte mit einer zufriedenstellenden Ausbeute von 61%. Zur Spaltung des Methylesters wurde ein besseres Protokoll verwendet, welches die freie Säure **107** in 86% Ausbeute lieferte.

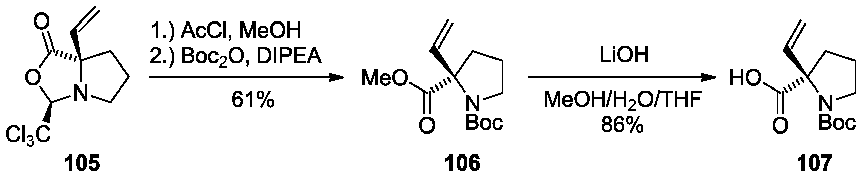

### Schema 44: Synthese des spiro-2-Vinylprolins 107.

Zur Synthese des zweiten Bausteins, einem *trans*-5-Vinylprolin-esters vom Typ **100** wurde auf eine in unseren Laboratorien entwickelte Synthese zurückgegriffen (Schema 45).

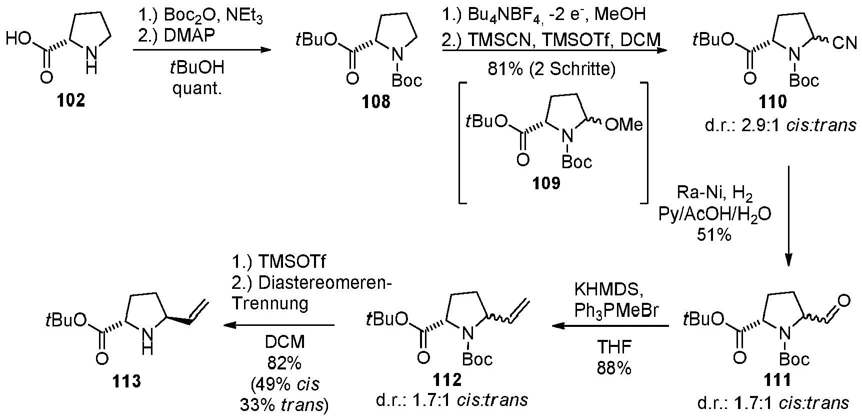

### Schema 45: Synthese der trans-5-Vinylprolinderivate.

Zunächst wurden 50 g L-Prolin **102** unter Verwendung von Boc₂O, Triethylamin und DMAP, C- und N-Termins in quanitativer Ausbeute geschützt. Die elektrochemische Oxidation des geschützten Prolins **108** konnte im 46 g (170 mmol) Maßstab mit einer Elektrodenfläche von 48x56 mm und 240 mA konstanter Stromstärke durchgeführt werden. Die Aufreinigung des Oxidationsproduktes führte zu hohen Ausbeuteverlusten, weshalb dieses direkt weiter umgesetzt und erst nach der Cyanierung zum Nitril **110** aufgereinigt wurde. Diese wurde in einer 1vol% Lösung von TMSOTf in DCM durch Zutropfen von 1.1 eq TMSCN durchgeführt und lieferte über zwei Stufen ein cis-trans-Isomerengemisch des Cyanids **110** mit einer Ausbeute von 81% (d.r.: 2.9:1, *cis: trans*). Die anschließende Hydrierung wurde im 18 g Maßstab bei 80 °C unter einer Wasserstoff Atmosphäre mit Raney-Nickel in einem Gemisch aus Pyridin, Essigsäure und Wasser (2:1:1) durchgeführt und lieferte den Aldehyd **111** in 51% Ausbeute. Die Verwendung einer Wasserstoffatmosphäre an Stelle von NaH₂PO₂ stellt eine klare Verbesserung der bekannten Methodik dar. Eine Wittig-Reaktion unter Nutzung von KHMDS als Base lieferte das gewünschte Produkt **112** in 88% Ausbeute erhalten (d.r.: 1.7:1, *cis:trans*). Entschützung mit TMSOTf in DCM und anschließende säulenchromatographische Diastereomerentrennung lieferte das benötigte *trans*-konfigurierte Prolin **113** in 33%, sowie das *cis-*Epimer **114** (nicht gezeigt) in 49% Ausbeute.

Mit dem so erhaltenen Material wurde die weitere Synthese der tricyclischen Diprolinmimetika ausgelotet (Peptidkupplung und nachfolgende Ringschlussmetathese). Trotz der sterischen Hinderung durch die beiden Vinylgruppen (besondes des spirocyclischen Bausteins **107**) gelang die Knüpfung der Peptidbindung unter Verwendung von HATU in NMP bei 85 °C. Dabei wurde die Carbonsäure **107** mit HATU präaktiviert, bevor die Zugabe des Amins **113** und Diisopropylethylamin als Base erfolgte. Die Identität des Kupplungsproduktes konnte im ¹H-NMR-Spektrum trotz Vorliegen eines Rotamerengemisches bestätigt werden.

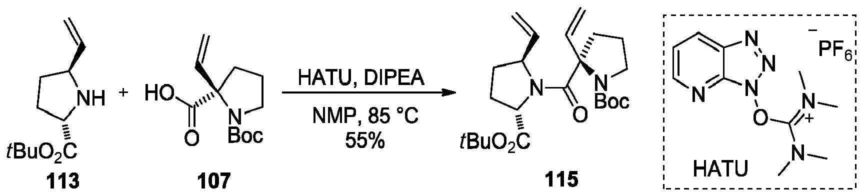

### Schema 46: Peptidkupplung der Vinylproline 113 und 107 unter Verwendung von HATU.

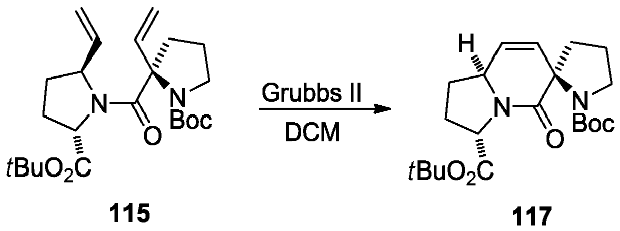

### Schema 53: Ringschlussmetathese zum Tricyclus 117.

Um den Tricyclus **117** durch Ringschlussmetathese zu erzeugen, wurde das Dipeptid **115** (Schema 53) zunächst mit dem Grubbs-II-Katalysator (30 mol-%) versetzt, und 7 Stunden in der Mikrowelle auf 50 °C erhitzt (300 W). Das gewünschte Produkt **117** wurde in 54% Ausbeuteerhalten. Außerdem konnten 22% Edukt **115** reisoliert werden. Der Erfolg der Ringschlussmetathese ließ sich dabei im ¹H-NMR-Spektrum eindeutig anhand der Änderung der Olefinsignale belegen. Desweiteren konnte die Struktur und Konfiguration des Tricyclus **117** mittels Röntgendiffraktometrie belegt werden.

Um den synthetisierten Tricyclus **117** für die Festphasen-Peptidsynthese nutzbar zu machen, mussten schließlich beide Schutzgruppen abgespalten und die Aminfunktion mit einer Fmoc-Schutzgruppe versehen werden. Diese ist die einzige N-Schutzgruppe von praktischer Bedeutung, die unter milden basischen Bedingungen abgespalten werden kann, wofür in der Regel sekundäre Amine wie Piperidin, Morpholin u.a. verwendet werden. Dadurch wird die Generierung der ungeschützten Aminofunktion bei der Festphasensynthese ohne zusätzliche Produktion von Salzen ermöglicht. Die erhöhte Basenlabilität beeinträchtigt den Einsatz der Fmoc-Gruppe in der organischen Synthese und erfordert deshalb den späten Wechsel der Schutzgruppe.

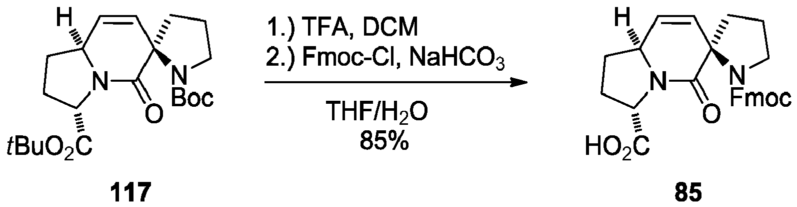

### Schema 54: Fmoc-Schützung zur Zielstruktur 85.

Zur gemeinsamen sauren Abspaltung der Boc-Schutzgruppe und des *tert*-Butylesters wurde **115** mit einem Überschuss Trifluoressigsäure (99%ig) in Dichlormethan versetzt und bei Raumtemperatur gerührt (Schema 54). Anschließend wurde der Rückstand unter Schotten-Baumann-Bedingungen in das Fmoc-geschützte Endprodukt **85** überführt. Dieses erwies sich als ausgesprochen polar, aber konnte dennoch mit Dichlormethan aus der wäßrigen Phase bei pH ≈ 1 extrahiert und anschließend säulenchromatographisch an Kieselgel aufgereinigt werden. Man erhielt **85** als amorphen altweißen Feststoff (85 %), dessen NMR-Spektren sich mit den Erwartungen deckten (s. Abbildung 15 für 600 MHz ¹H-NMR-Spektrum (MeOH-d⁴), 60:40 Rotamerengemisch).

### Biologische Ergebnisse

Diese Ergebnisse sind insofern besonders bemerkenswert, da es sich hierbei um bisher für die EVH1-Domäne nicht beschriebene Experimente handelt und diese zu einer Reihe wichtiger Erkenntnisse führen, die wie folgt zusammengefasst werden können und eine Grundlage für zukünftige Forschungsarbeiten bilden:
1) Der Einbau des synthetisch hergestellten Tricyclus **85** in verschiedene Testpeptide unter den Standard-Kupplungsbedingungen (DIC/HOBt) der Festphasen-Peptidsynthese verlief ohne Probleme.
2) Die bisher getroffenen Voraussagen, dass sich die mittleren beiden Prolin-Positionen des F/WPPPP-Kernmotivs ersetzen lassen sollten, konnten bestätigt werden.
3) Die Vorhersage der Reaktivkonformation bei Bindung der PPII-Helix an den EVH1-Rezeptor wird durch die gute Bindungsaffinität des Ligandenpeptids **I** bestätigt, bzw. es stimmen die biologisch benötigte Konformation und die tatsächliche Konformation des Tricyclus **85** gut überein.
4) Der erwartete günstige Entropie-Effekt durch lokale, konformationelle Vorfixierung des Liganden scheint sich durch die festgestellte höhere Bindungsaffinität im Vergleich zur nativen Peptidsequenz zu bestätigen. Andererseits verhindert die geometrische Fixierung aber auch nicht die Annäherung von Ligand und Domänenprotein und damit die Bindungsbildung.

### Erste biologische Ergebnisse und Ausblick

Mit Hilfe der synthetischen Verbindung **85** wurden beispielhaft Ligandenpeptide mit teilweise oder vollständig ersetzten Prolinsequenzen synthetisiert und auf ihre Wechselwirkung mit der EVH1-Domäne untersucht (*p* = Baustein **85).** Dabei zeigte sich, dass durch den Einbau des Mimetikums **85** die Bindungsaffinität der Ligandenpeptide zur Domäne gesteigert werden konnte. Der Austausch aller Proline durch miteinander verknüpfte Bausteine von 85 und dem PPII-mimetischen Baustein **86** führte zu Ligandenpeptiden mit einer deutlich verbesserter Affinität im Vergleich zu der des nativen Peptides.

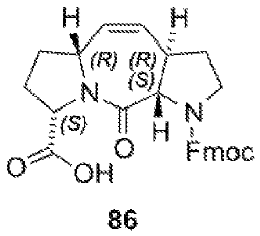

Dies ist in der nachfolgenden Tabelle 1 zusammengefasst, die die Bindungsaffinitäten der Ligandenpeptide sowie der zu Grunde liegenden nativen Peptidliganden auflistet.

**Tabelle 1**

| Ligand | K_{D} (Fluoreszenztitration) | K_{D} (ITC) |
|---|---|---|
| Ac-SFE-[2-Cl-F]-PPPPTEDEL-NH₂ | 5 µM | 2 µM |
| Ac-SFE-[2-CI-F]-*px*TEDEL-NH₂ (*p*: *Baustein 85; x:Baustein 86* | 220 nM | 330 nM |

Die Bindungsaffinitäten wurden mittels Fluoreszenztitration und isothermaler Calorimetrie bestimmt. Da das generelle Konzept in Hinblick auf die Synthese und den biologischen Einsatz der Zielstruktur **85** erfolgreich umgesetzt werden konnte, eröffnen sich auf dieser vielversprechenden Grundlage zahlreiche weitere Möglichkeiten, mit Hilfe kleiner synthetischer Moleküle gezielt relevante Proteindomänen zu adressieren, die polyprolinhaltige Liganden binden. Neben der Variation bestehender Molekülstrukturen wäre auch eine Ausweitung des Konzepts auf die Synthese von Liganden für andere Domänen (Mena-, ena- und evl-EVH1, WW, SH3 u.a.), die Kombination mit anderen Prolin-minetischen Scaffolds sowie der volständige Ersatz von poly-Prolinmotiven durch Aneinanderreihung von **85** denkbar.

### Experimenteller Teil

Glasapparaturen wurden dreimal an einer Vakuum/Argon-Doppelhahnapparatur im Ölpumpenvakuum mit einem Enddruck von 0,2-2 mbar evakuiert, mit einem Propangasbrenner ausgeheizt und anschließend mit Argon belüftet. Einwegspritzen, Kanülen und Transferkanülen wurden acetonnass im Trockenschrank bei 80 °C gelagert. Lösungsmittel wurden an einem Vakuumrotationsverdampfer der Firma Büchi bei 1013 - 10 mbar Druck und 40 °C Wasserbadtemperatur entfernt.

THF, Toluol und Diethylether wurden unter Argon über Natrium mit Benzophenon als Indikator, Chloroform über Phosphorpentoxid und Dichlormethan über Calciumhydrid, mehrere Stunden refluxiert und anschließend destilliert. Methanol wurde über Magnesium und Iod refluxiert und anschließend destilliert. THF, Methyl- THF, Toluol, Diethylether und Dichlormethan wurden vor Verwendung frisch destilliert. Nicht absolutierte Lösungsmittel wurden ebenfalls vor Verwendung destilliert. Übrige Reagenzien wurden von den Firmen ABCR, Acros, Aldrich, Chemetall, Fluka, Merck, Lancaster, Riedel-de Haen, und Strem bezogen und ohne weitere Reinigung eingesetzt.

Säulenchromatographische Trennungen wurden an Kieselgel 60 (230-400 mesh, Merck) oder Aluminiumoxid (50-200 µm, Brockmann I, Acros) durchgeführt. Methanol, Dichlormethan, Ethylacetat und Cyclohexan wurden vor Gebrauch destilliert. Laufmittelgemische sind in Volumenanteilen angegeben.

IR-Spektren wurden bei Raumtemperatur an einem FT-IR-Paragon 1000 Gerät der Firma Perkin-Elmer mit ATR-Technik auf einem ZnSe-Kristall, auf den die Substanzen als Lösungen aufgetragen wurden, aufgenommen. Absorptionen sind in Wellenzahlen ^{∼}v in cm-1 angegeben und mit s (strong), m (medium), w (weak) gekennzeichnet. Gegebenenfalls ist dem ein br (breites Signal) beigefügt.

Methanol für präparative Elektrolysen wurde vor Verwendung destilliert. Als Spannungsquelle diente ein lineares Labornetzgerät VLP-1602 Pro der Firma Voltcraft und 48x28 mm Graphit-Plattenelektroden der Firma Didac-Tec.

Schmelzpunkte wurden mit einem Melting Point B-545 der Firma Büchi und einem Mikro-Heiztisch System PolyTherm A der Firma Wagner und Munz bestimmt.

¹H- und ¹³C-NMR-Spektren wurden bei Raumtemperatur an den Geräten Avance DPX 300, AC 300, AV 400, Avance DPX 500 und AV 600 der Firma Bruker aufgenommen. Chemische Verschiebungen sind in ppm relativ zu den Signalen der verwendeten Lösungsmittel (¹³C-NMR) bzw. nicht-deuterierter Spuren dieser Lösungsmittel (¹H-NMR) angegeben (CHCl₃) = 7.24 ppm, (CDCl₃) = 77.00 ppm, (CD₂HOD) = 3.31 ppm, (MeOD) = 49.05 ppm.
In ¹H-Spektren sind Multiplizitäten mit:
s = Singulett
d = Dublett
t = Triplett
q = Quadroplett
sept = Septett
m = Multiplett
beschrieben.
Die ¹³C-Kernresonanzspektren wurden als APT aufgenommen und mit
s = quartäres C-Atom
d = tertiäres C-Atom
t = sekundäres C-Atom
q = primäres C-Atom
beschrieben. Kopplungskonstanten J sind in Hertz angegeben. Zuordnungen der NMR-Signale wurden mit H,H-COSY, TOCSY, HMBC und HMQC-Spektren belegt. Die für die Zuordnung gewählte Nummerierung der Kohlenstoffatome ist den jeweiligen Abbildungen zu entnehmen und stimmt nicht notwendigerweise mit der ILTPAC-Nomenklatur überein.

Die DC wurde an Merck Folien HX616606 (20x20 mm, 0.2 mm Schichtdicke) Folien durchgeführt. Alle Laufmittelgemische sind in Volumenteilen angegeben. Anfärbung erfolgte mittels UV-Lampe, Kaliumpermanganatlösung oder Cer(IV)sulfatlösung und anschließendem Erhitzen mit einem Heißluftfön.

Low Resolution (LR) MS-Spektren wurden an einem Finnigan MAT Incos 50 Galaxy System (GC/DIP-MS) mit einer Optima 5-Kapillarsäule und Wasserstoff als Trägergas und einem kombinierten HP6890/MSD5973 (GC-MS) System der Firma Agilent Technologies und einer HP5 Kapillarsäule aufgenommen. Die Ionisierung der Probe erfolgt per EI bei 70 eV. Die Fragmente wurden in m/z angegeben, die Intensität der Signale bezieht sich auf den intensivsten Peak mit 100%.

High-Resolution (HR) MS-Spektren wurden an einem Finnigan MAT HSQ-30 durch DIP-Methode gemessen. Die Ionisierung erfolgte bei 70 eV nach dem EI- oder ESIVerfahren.

Die Drehwerte wurden in einer Küvette mit einer Länge von 10 cm an einem Polarimeter 343 der Firma Perkin-Elmer gemessen. Die Konzentrationen sind in g □ (100 ml)⁻¹ angegeben.

Röntgen-Kristallstrukturanalysen wurden an einem Nonius Kappa CCD Diffraktometer durchgeführt.

### (3R, 7aS)-3-(Trichloromethyl)-tetrahydropyrrolo[1,2-c]oxazol-1(3H)-on (103)

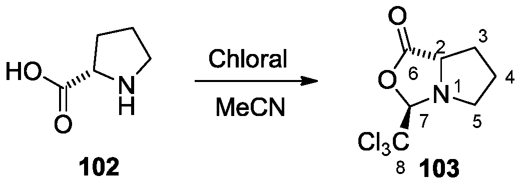

Zu einer Suspension von 10.470 g (90.94 mmol, 1.0 eq) *L*-Prolin (**102**) in 45 ml MeCN wurden bei RT 22 ml (225 mmol, 2.5 eq) Chloral zugegeben und die entstehende Lösung 19.5 h bei RT gerührt. Das Lösungsmittel wurde bei vermindertem Druck entfernt, der Rückstand in 100 ml DCM gelöst und filtriert. Das Filtrat wurde eingeengt und dieser Ablauf weitere dreimal wiederholt. Es wurden 18.141 g (74.20 mmol, 82%) des Produktes **103** als weißer Feststoff erhalten.

**M (C₇H₈Cl₃NO₂):** 244.50 g mol⁻¹.

**[α]²⁰_{D} :** 34.3 ° (c = 0.970, Benzol).

**Schmelzpunkt:** 109.6 °C.

**¹H-NMR (400 MHz, CDCl₃):** δ = 1.78-1.64 (m, 1H, H4'), 1.96-1.85 (m, 1H, H4), 2.13-2.02 (m, 1H, H3'), 2.26-2.13 (m, 1H, H3), 3.15-3.06 (m, 1H, H5'),3.39 (ddd, *J* 10.9, 7.8, 6.0, 1H, H5), 4.09 (dd, *J* 8.9, 4.6, 1H, H2), 5.14 (s, 1H, H7).

**¹³C-NMR (100 MHz, CDCl₃:** δ = 25.32 (t, C4), 29.91 (t, C3), 57.87 (t, C5), 62.38 (t, C2), 100.62 (s, C8), 103.60 (d, C7), 175.43 (s, C6).

**IR (FT-ATR):** ^{∼}v = 2958 (m), 2920 (w), 2893 (w), 2866 (w), 1784 (s), 1725 (m), 1449 (w), 1373 (w), 1359 (w), 1322 (m), 1283 (m), 1270 (m), 1243 (m), 1174 (s), 1107 (s), 1083 (m), 1043 (w), 1001 (s), 958 (s), 913 (w), 898 (m), 838 (s), 813 (s), 790 (s), 744 (s).

### Kristalldaten

| | | |
|---|---|---|
| Identification code | z2 | |
| Empirical formula | C7 H8 Cl3 N 02 | |
| Formula weight | 244.49 | |
| Temperature | 100(2) K | |
| Wavelength | 0.71073 A | |
| Crystal system, space group | Orthorhombic, P212121 | |
| Unit cell dimensions | a = 8.5642(6) A | alpha = 90 deg. |
| | b = 8.8955(4) A | beta = 90 deg. |
| | c = 12.9294(11) A | gamma = 90 deg. |
| Volume | 985.00(12) A^3 | |
| Z, Calculated density | 4, 1.649 Mg/m^3 | |
| Absorption coefficient | 0.895 mm^-1 | |
| F(000) | 496 | |
| Crystal size | .3 x .3 x .2 mm | |
| Theta range for data collection | 2.78 to 26.99 deg. | |
| Limiting indices | -8<=h<=10 -9<=k<=11 -16<=I<=10 | |
| Reflections collected / unique | 3966 / 2109 [R(int) = 0.0254] | |
| Reflection observed [I>2sigma(I)] | 1902 | |
| Completeness to theta = 26.99 | 99.9 % | |
| Absorption correction | None | |
| Refinement method | Full-matrix least-squares on F^2 | |
| Data / restraints / parameters | 2109 / 0 /150 | |
| Goodness-of-fit on F^2 | 1.020 | |
| Final R indices [I>2sigma(I)] | R1 = 0.0292, wR2 = 0.0547 | |
| R indices (all data) | R1 = 0.0354, wR2 = 0.0562 | |
| Absolute structure parameter | -0.04(5) | |
| Largest diff. peak and hole | 0.258 and -0.354 e.A^-3 | |

### (3R,7aR)-1-Oxo-3-(trichloromethyl)-hexahydropyrrolo[1,2-c]-oxazol-7a carbaldehyd (104)

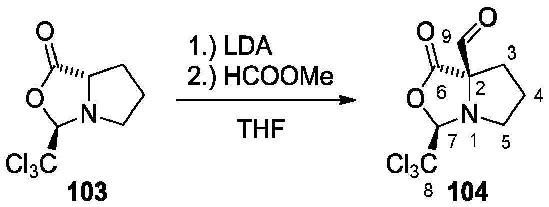

Zu einer Lösung von 14.092 g (57.64 mmol, 1.0 eq) des geschützten Prolins **103** wurden nach Zugabe von etwa 3.5 g LiCl bei -78 °C 138 ml (86.46 mmol, 1.5 eq, 0.625 M) LDA in THF über 60 min zugetropft. Die Lösung wurde 30 min bei dieser Temperatur gerührt und dann wurden sehr langsam 15 ml (241.85 mmol, 4.0 eq) HCOOMe über 120 min zugetropft. Die Lösung wurde weitere 30 min bei -78 °C gerührt und dann über 1 h auf -40 °C gebracht. Dann wurden 120 ml einer 10%igen Zitronensäurelsg. in Wasser zugegeben, dreimal mit je 120 ml MTBE extrahiert, die vereinigten organischen Phasen mit 500 ml ges. NaCl-Lsg. gewaschen, über Magnesiumsulfat getrocknet und das Lösungsmittel bei vermindertem Druck entfernt. Durch Chromatographie an Kieselgel mit EtOAc/CyHex 1:4 als Laufmittel wurden 10.388 g (38.12 mmol, 66%) des Produktes **104** als weißer Feststoff erhalten.

**M (C₈H₈Cl₃NO₃):** 272.51 g mol⁻¹.

**DC:** R_{f} = 0.13 (EtOAc/CyHex 1:4).

**[α]²⁵_{D} :** 29.9 ° (c = 0.520, CHCl₃).

**Schmelzpunkt:** 84.8 °C.

**¹H-NMR (300 MHz, CDCl₃):** δ = 2.00-1.77 (m, 2H, H4), 2.44-2.20 (m, 2H, H3), 3.31 (dt, *J* 11.6, 6.0, 1H, H5'), 3.52 (ddd, *J* 11.4, 7.7, 6.3, 1H, H5), 5.16 (s, 1H, H7), 9.59 (s, 1H, H9).

**¹³C-NMR (75 MHz, CDCl₃:** δ = 25.47 (t, C4), 33.84 (t, C3), 58.94 (t, C5), 78.01(s, C2), 99.92 (s, C8), 102.36 (d, C7), 169.27 (s, C6), 193.43 (d, C9).

**GC-MS:** m/z = 272 ([M]⁺, 1), 242 ([M]⁺-CHO, 20), 117 (24), 96 (59), 68 (35), 41 (100).

**IR (FT-ATR):** ^{∼}v = 3017 (w), 2914 (w), 2846 (w), 1807 (m), 1729 (w), 1456 (w), 1353 (w), 1319 (w), 1272 (w), 1213 (m), 1190 (w), 11343 (w), 1107 (w), 1058 (w), 1020 (w), 993 (w), 972 (w), 929 (w), 839 (w), 816 (w), 747 (s), 667 (m).

### Kristalldaten

| | | |
|---|---|---|
| Identification code | zz | |
| Empirical formula | C8 H8 Cl3 N 03 | |
| Formula weight | 272.50 | |
| Temperature | 100(2) K | |
| Wavelength | 0.71073 A | |
| Crystal system, space group | Triclinic, P1 | |
| Unit cell dimensions | a = 6.0569(8) A | alpha = 70.252(7) deg. |
| | b = 6.9158(10) A | beta = 87.601 (10) deg. |
| | c = 7.9722(10) A | gamma = 64.141 (9) deg. |
| Volume | 280.63(7) A^3 | |
| Z, Calculated density | 1, 1.612 Mg/m^3 | |
| Absorption coefficient | 0.801 mm^-1 | |
| F(000) | 138 | |
| Crystal size | .2 x .2 x .07 mm | |
| Theta range for data collection | 2.74 to 26.99 deg. | |
| Limiting indices | -7<=h<=6 -8<=k<=7 -10<=I<=9 | |
| Reflections collected / unique | 1616 / 1616 [R(int) = 0.0000] | |
| Reflection observed [I>2sigma(I)] | 1538 | |
| Completeness to theta = 26.99 | 95.6 % | |
| Absorption correction | None | |
| Refinement method | Full-matrix least-squares on F^2 | |
| Data / restraints / parameters | 1616 / 3 /168 | |
| Goodness-of-fit on F^2 | 1.051 | |
| Final R indices [I>2sigma(I)] | R1 = 0.0241, wR2 = 0.0573 | |
| R indices (all data) | R1 = 0.0262, wR2 = 0.0583 | |
| Absolute structure parameter | 0.02(5) | |
| Largest diff. peak and hole | 0.202 and -0.239 e.A^-3 | |

### (3R,7aR)-3-(Trichloromethyl)-7a-vinyltetrahydropyrrolo[1,2-c]oxazol-1-(3H)-on (105)

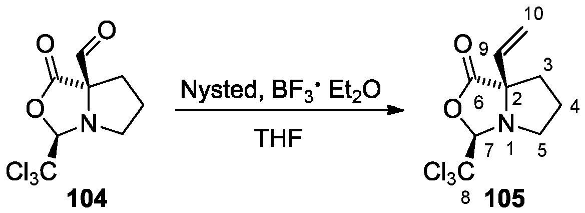

Zu einer Suspension von 97 g (42.23 mmol, 2.5 eq) Nysted-Reagenz (50w% in THF, Aldrich) in 130 ml THF wurden bei 0 °C erst 6 ml (42.23 mmol, 2.5 eq) BF₃ Et₂O in 50 ml THF über 20 min und dann 4.603 g (16.89 mmol, 1.0 eq) des Eduktes **104** in 30 ml THF zugetropft. Das Reaktionsgemisch wurde 2 h bei RT gerührt und nach Zugabe von 200 ml 1 N HCl-Lsg. dreimal mit je 200 ml n-Hexan extrahiert. Die vereinigten organischen Phasen wurden über Magensiumsulfat getrocknet, das Lösungsmittel bei vermindertem druck entfernt und durch Chromatographie an Kieselgel mit EtOAc/CyHey 1:10 als Laufmittel 3.051 g (11.28 mmol, 67%) des Produktes **105** als farbloses Öl gewonnen.

**M (C₉H₁₀Cl₃NO₂):** 270.54 g mol⁻¹.

**DC:** R_{f} = 0.17 (EtOAc/CyHex 1:10).

**[α]²²_{D} :** 49.9 ° (c = 0.660, CHCl3).

**Schmelzpunkt:** 84.8 °C.

**¹H-NMR (300 MHz, CDCl₃):** δ = 2.00-1.77 (m, 2H, H4), 2.44-2.20 (m, 2H, H3), 3.31 (dt, *J* 11.6, 6.0, 1H, H5'), 3.52 (ddd, *J* 11.4, 7.7, 6.3, 1H, H5), 5.16 (s, 1H, H7), 9.59 (s, 1H, H9).

**¹³C-NMR (75 MHz, CDCl₃:** δ = 25.47 (t, C4), 33.84 (t, C3), 58.94 (t, C5), 78.01 (s, C2), 99.92 (s, C8), 102.36 (d, C7), 169.27 (s, C6), 193.43 (d, C9).

**GC-MS:** m/z = 272 ([M]⁺, 1), 242 ([M]⁺-CHO, 20), 117 (24), 96 (59), 68 (35), 41 (100).

**IR (FT-ATR):** ^{∼}v = 3017 (w), 2914 (w), 2846 (w), 1807 (m), 1729 (w), 1456 (w), 1353 (w), 1319 (w), 1272 (w), 1213 (m), 1190 (w), 11343 (w), 1107 (w), 1058 (w), 1020 (w), 993 (w), 972 (w), 929 (w), 839 (w), 816 (w), 747 (s), 667 (m).

### (R)-1-tert-Butyl-2-methyl-2-vinylpyrrolidin-1,2-dicarboxylat (106)

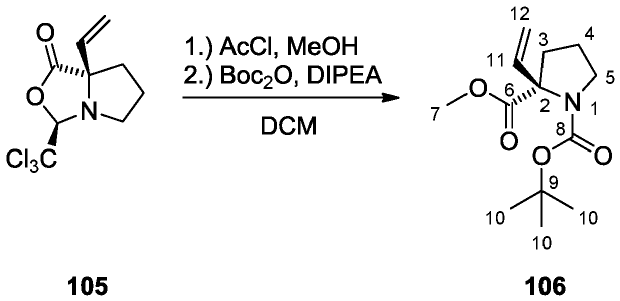

Zu 60 ml MeOH wurden bei 0 °C zunächst 15 ml (212.24 mmol, 10 eq) AcCl und anschließend eine Lösung von 5.763 g (21.30 mmol, 1.0 eq) des Eduktes **105** in 20 ml MeOH zugegeben. Die Lösung wurde 7 Tage bei RT gerührt, das Lösungsmittel bei vermindertem Druck entfernt, der Rückstand in 20 ml MeOH aufgenommen und das Lösungsmittel erneut bei vermindertem Druck entfernt. Dann wurde der Rückstand in 60 ml DCM aufgenommen, 17.20 ml (106.50 mmol, 5.0 eq) DIPEA zugegeben und anschließend eine Lösung von 17.7 ml (82.72 mmol, 3.9 eq) Boc₂O in 35 ml zugetropft. Es wurde 2.5 Tage bei RT gerührt, das Lösungsmittel bei vermindertem Druck entfernt und der Rückstand in 100 ml MTBE aufgenommen. Die Etherphase wurde nacheinander mit je 100 ml 1 N HCl-Lsg., ges. NaHCO₃-Lsg. und ges. NaCl-Lsg. gewaschen, über Magnesiumsulfat getrocknet, das Lösungsmittel bei vermindertem Druck entfernt und nach Chromatographie an Kieselgel mit EtOAc/CyHex 1:6 als Laufmittel wurden 3.654 g (14.31 mmol, 67%) des geschützten Vinylprolins 106 als farbloses Öl erhalten.

**M (C₁₃H₂₁NO₄):** 255.31 g mol⁻¹.

**DC:** R_{f} = 0.20 (EtOAc/CyHex 1:4).

**[α]²²_{D} :** 62.5 ° (c = 0.455, CHCl₃).

**¹H-NMR (300 MHz, CDCl₃):** δ = 1.38 (ψ-d, *J* 28.5(ψ), 9H, H10), 1.90-1.74 (m, 2H, H4), 2.03-1.91 (m, 1H, H3'), 2.25-2.08 (m, 1H, H3), 3.67-3.46 (m, 2H, H5), 3.71 (ψ-d, *J* 1.7(ψ), 3H, H7), 5.02 (ψ-dd, *J* 17.0, 4.6(ψ), 1H, H12'), 5.13 (ψ-dd, *J* 10.5, 4.2(ψ), 1H, H12), 6.30 (ψ-ddd, *J* 17.2, 10.5, 3.8(ψ), 1H).

**¹³C-NMR (75 MHz, CDCl₃:** δ = 22.76/21.87 (t, C4), 28.38/28.18 (q, C10), 39.19/37.93 (t, C3), 47.99/47.82 (t, C5), 52.39/52.20 (q, C7), 69.45/69.30 (s, C2), 79.97/79.72 (s, C9), 113.08/112.96 (t, C12), 137.15/136.39 (d, C11), 153.55/153.44 (s, C8), 173.72 (s, C6).

**GC-MS:** m/z = 196 ([M]⁺-C₂H₃O₂, 13), 140 (75), 96 (63), 56 (100).

IR **(FT-ATR):** ^{∼}v = 3085 (w), 2974 (m), 2876 (w), 1742 (s), 1696 (s), 1641 (w), 1476 (w), 1453 (w), 1432 (w), 1387 (s), 1364 (s), 1289 (w), 1258 (s), 1214 (m), 1165 (s), 1122 (s), 1071 (m), 1021 (w), 989 (w), 959 (w), 933 (w), 921 (w), 881 (w), 857 (w), 784 (w), 770 (w), 741 (w), 663 (w).

**HR-MS:** (ESI, C₁₃H₂₁NNaO₄): 278.137 ± 0.003 u (278.1368 u).

### (R)-1-(tert-Butoxycarbonyl)-2-vinylpyrrolidin-2-carbonsäure (107)

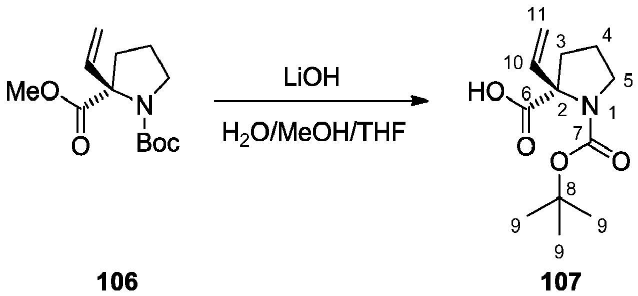

Eine Lösung von 858 mg (3.36 mmol, 1.0 eq) des Eduktes **106** in 3.4 ml (6.72 mmol, 2.0 eq, 2 N) wässriger LiOH-Lsg., 3.4 ml MeOH und 10.2 ml THF (1:1:3) wurde 5 h bei 50 °C gerührt. Der pH-Wert wurde kontrolliert (pH = 10), das Reaktionsgemisch zweimal mit MTBE gewaschen und die wässrige Phase bei 0 °C durch Zugabe von 1 N HCl-Lsg. auf pH = 1 gebracht. Dann wurde dreimal mit DCM extrahiert, die vereinigten DCM-Phasen über Magnesiumsulfat getrocknet und das Lösungsmittel bei vermindertem Druck entfernt. Es wurden 700 mg (2.90 mmol, 86%) des Produktes **107** als weißer Feststoff erhalten.

**M (C₁₂H₁₉NO₄):** 241.28 g mol⁻¹.

**[α]²³_{D} :** -25.8 ° (c = 0.490, CHCl₃).

**Schmelzpunkt:** 134.4 °C.

**¹H-NMR (300 MHz, CDCl₃):** δ = 1.34 (ψ-d, *J* 29.3(ψ), 9H, H9), 2.04-1.68 (m, 3H, H4, H3'), 2.42-2.13 (m, 1H, H3), 3.66-3.32 (m, 2H, H5), 5.20-4.87 (m, 2H, H11), 6.29-6.00 (m, 1H, H10), 10.22 (s, 1H, COOH).

**¹³C-NMR (75 MHz, CDCl₃:** δ = 21.71/22.56 (t, C4), 27.93/28.19 (q, C9), 37.82/39.16 (t, C3), 47.68/48.26 (t, C5), 69.18/70.15 (s, C2), 80.51/80.81 (s, C8), 113.02/114.02 (t, C11), 136.15/136.48 (d, C10), 153.53/154.84 (s, C7), 175.90/178.08 (s, C6).
**IR (FT-ATR):** ^{∼}v = 2968 (m, br), 2875 (m), 2620 (w), 1727 (s), 1690 (w), 1634 (s), 1550 (w), 1475 (m), 1449 (m), 1418 (s), 1393 (s), 1366 (s), 1349 (m), 1248 (s), 1214 (w), 1158 (s), 1122 (m), 1078 (m), 1013 (w), 996 (w), 982 (m), 942 (m), 884 (m), 852 (s), 774 (s), 755 (w), 719 (s).

**HR-MS:** (ESI, C₁₂H₁₉NNaO₄) 264.121 ± 0.003 u (264.1212 u).

### (S)-Di-tert-butyl-pyrrolidin-1,2-dicarboxylat (108)

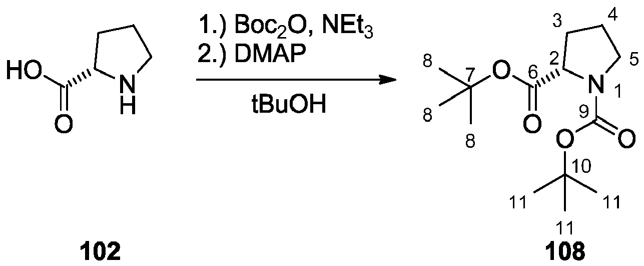

Zu 10.1 g (87.73 mmol, 1.0 eq) L-Prolin **(102)** in 180 ml tBuOH und 3.70 ml (26.32 mmol, 0.3 eq) NEt₃ wurden 52 ml (251 mmol, 2.7 eq) Boc₂O gegeben und 4 h bei RT gerührt bis die Lösung klar war. Dann wurden 3.295 g (26.32 mmol, 0.3 eq) DMAP dazugegeben und die Lösung im Eisbad bis zum beginnenden Erstarren gekühlt. Es wurde 17 h bei RT gerührt und die Lösung 1.5 h unter starkem Rühren auf 45 °C erhitzt bis keine Gasentwicklung mehr beobachtet wurde. Der Ansatz wurde mit 450 ml MTBE versetzt, nacheinander mit je 300 ml 1N HCl-Lsg., gesättigter NaHCO₃-Lsg. und gesättigter NaCl-Lsg. gewaschen, die organische Phase über Magnesiumsulfat getrocknet und das Lösungsmittel bei vermindertem Druck entfernt. Es wurden 23.8 g (87.70 mmol, 99%) des geschützten Prolins **108** als farbloses Öl erhalten. Für analytische Zwecke wurde ein kleiner Teil des Produktes durch Chromatographie an Kieselgel mit EtOAc/CyHex (1:9) als Laufmittel aufgereinigt.

**M (C₁₄H₂₅NO₄):** 271.35 g mol⁻¹.

**DC:** R_{f} = 0.12 (EtOAc/CyHex 1:9).

**[α]²⁰_{D} :** -53.1 ° (c = 0.670, CHCl₃).

**¹H-NMR (300 MHz, CDCl₃):** δ = 1.51-1.25 (m, 18H, H8, H11), 1.94-1.67 (m, 3H, H4, H3), 2.24-1.94 (m, 1H, H3'), 3.54-3.17 (m, 2H, H5), 4.10 (ψ-ddd, *J* 25.5(ψ), 8.6, 2.9, 1H, H2).

**¹³C-NMR (75 MHz, CDCl₃:** δ = 24.05/23.26 (t, C4), 28.21/27.84 (q, C8, C11), 30.74/29.73 (t, C3), 46.35/46.15 (t, C5), 59.52 (d, C2), 79.40/79.20 (s, C10), 80.61 (s, C7), 154.14/153.79 (s, C9), 172.12 (s, C6).

**GC-MS:** m/z = 271 ([M]⁺, 1), 170 ([M]⁺-C₅H₉O₂, 15), 142 (9), 114 (95), 57 (100), 19 (26).

**IR (FT-ATR):** ^{∼}v = 2973 (s), 2928 (m), 2880 (w), 1738 (s), 1697 (s), 1476 (m), 1454 (m), 1396 (s), 1364 (s), 1289 (m), 1252 (s), 1215 (s), 1148 (s), 1085 (s), 1028 (w), 978 (m), 939 (m), 917 (m), 895 (m), 851 (m), 840 (m), 797 (w), 770 (m).

### (S)-Di-tert-butyl-5-methoxypyrrolidin-1,2-dicarboxylat (109)

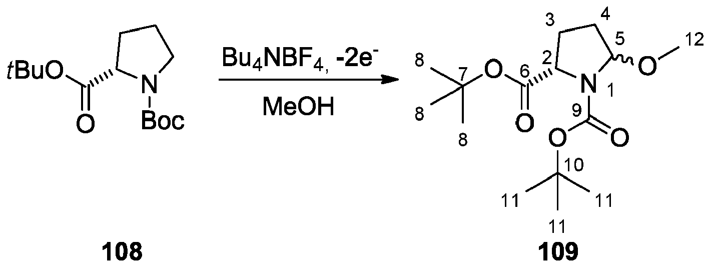

In eine Lösung von 46.129 g (170.00 mmol) des geschützten Prolins 108 (0.5 M) und 5.6 g (17 mmol) Bu₄NBF₄ (0.05 M) in 340 ml Methanol wurden bei 0 °C zwei Graphit-Plattenelektroden (48x56 mm) im Abstand von 5 cm eingetaucht und bei 240 mA konstanter Stromstärke elektrolysiert. Nach dem Durchgang von 2.46 F mol⁻¹ wurde das Lösungsmittel bei vermindertem Druck entfernt, und mit 1000 ml EtOAc/CyHex (1:1) als Laufmittel über Kieselgel filtriert. Das Lösungsmittel wurde bei vermindertem Druck entfernt und 50.624 g (167.97 mmol, 99%) Rohprodukt 109 als klares Öl erhalten und ohne weitere Aufreinigung weiter umgesetzt. Für analytische Zwecke wurde ein kleiner Teil des Produktes durch Chromatographie an Kieselgel mit EtOAc/CyHex (1:7) als Laufmittel aufgereinigt.

**M (C₁₅H₂₇NO₅)**: 301.38 g mol⁻¹.

**DC:** R_{f} = 0.43 (EtOAc/CyHex 1:7).

**¹H-NMR (300 MHz, CDCl₃):** δ = 1.59-1.23 (m, 18H, H8, H11), 2.45-1.62 (m, 4H, H3, H4), 3.63-3.20 (m, 3H, H12), 4.29-3.96 (m, 1H, H2), 5.34-4.99 (m, 1H, H5).

**¹³C-NMR (75 MHz, CDCl₃:** δ = 28.23/28.15/27.92/27.82 (C8, C11), 30.82/29.79 (t, C3), 32.89/32.17 (t, C4), 32.89/32.17 (t, C4), 60.24/59.93/59.83/59.62 (d, C2), 80.90/80.69/80.36/80.27 (s, C7, C10), 89.22/88.34 (d, C5), 154.43/154.21/153.81 (s, C9), 171.73/171.67/171.59 (s, C6).

**GC-MS:** m/z = 301 ([M]⁺, 1), 200 ([M^{]+}-C₅H₉O₂, 22), 172 (8), 144 (32), 100 (80), 57 (100), 29 (14).

**IR (FT-ATR):** ^{∼}v = 2975 (s), 2928 (s), 2825 (w), 1738 (s), 1699 (s), 1476 (m), 1455 (m), 1364 (s), 1327 (s), 1252 (s), 1157 (s), 1084 (s), 1024 (m), 987 (s), 939 (s), 911 (s), 885 (s), 841 (s), 797 (s), 772 (s), 729 (m).

### (S)-Di-tert-butyl-5-cyanopyrrolidin-1,2-dicarboxylat (110)

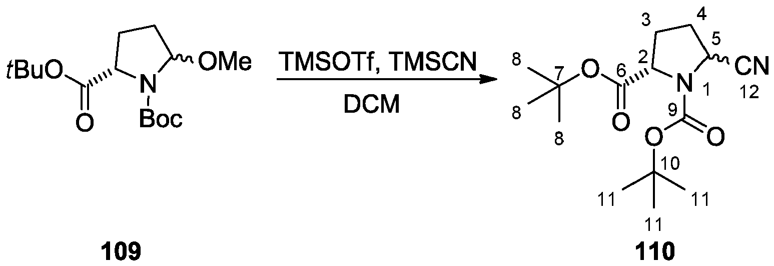

Zu 50.624 g (167.97 mmol, 1.0 eq) des rohen Prolins 109 in 440 ml trockenem DCM wurden bei -40 °C erst 4.4 ml TMSOTf (1vol%), dann 25.8 ml (191.81 mmol, 1.1 eq) TMSCN zugetropft und 1.5 h bei dieser Temperatur gerührt. Die Lösung wurde mit 65 ml MeOH versetzt, das Lösungsmittel bei vermindertem Druck entfernt und durch Chromatographie an neutralem Aluminiumoxid mit EtOAc/CyHex 1:9 als Laufmittel 40.569 g (136.89 mmol, 81% über zwei Schritte) eines Diastereomerengemisches des Nitrils **110** erhalten (d.r.: 3:1 *cis: trans*). Für analytische Zwecke wurden ein kleiner Teil der einzelnen Isomere chromatographisch getrennt.

**M (C₁₅H₂₄N₂O₄)**: 296.36 g mol-1.

**trans-DC:** R_{f} = 0.33 (EtOAc/CyHex 1:9).

**trans- [α]²⁰_{D} :** -88.6 °(c = 0.550, CHCl₃).

**trans-Schmelzpunkt:** 106.3 °C.

**trans-¹H-NMR (300 MHz, CDCl₃):** δ = 1.48-1.32 (m, 18H, H8, H11), 2.53-1.97 (m, 4H, H3, H4), 4.20 (Ψ-dd, *J* 26.2(Ψ), 7.9 1H, H2), 4.61 (Ψ-dd, *J* 30.2(Ψ), 7.2, 1H, H5).

**trans⁻¹³C-NMR (75 MHz, CDCl₃:** δ = 28.04/27.81 (q, C8, C11), 29.60/28.59/28.46 (t, C3, C4), 47.59/47.48 (d, C5), 59.52/59.41 (d, C2), 81.84/81.78/81.70/81.49 (s, C7, C10), 118.82/118.75 (s, C12), 152.77/152.48 (s, C9), 170.79/170.70 (s, C6).

**trans-GC-MS:** m/z = 255 ([M]⁺, 1), 199 (5), 154 ([M]⁺-C₅H₉O₂, 100).
**trans-IR (FT-ATR):** ~v = 2973 (m), 2928 (w), 2238 (w), 1734 (s), 1704 (s), 1473 (w), 1455, 1364 (s), 1323 (m), 1306 (m), 1252 (m), 1225 (s), 1144 (s), 1119 (s), 1061 (m), 1034 (w), 987 (w), 914 (m), 842 (m), 820 (w), 773 (m), 756 (w).

**trans-HR-MS**: (ESI, C₁₅H₂₄N₂NaO₄): 319.163 ± 0.001 u (319.1634 u).

**cis-DC:** R_{f} = 0.27 (EtOAc/CyHex 1:9).

**cis- [α]²⁰D :** 19.2 °(c = 0.420, CHCl₃).

**cis-Schmelzpunkt:** 81.3 °C.

**cis-¹H-NMR (300 MHz, CDCl₃):** δ = 1.63-1.33 (m, 18H, H8, H10), 2.47-2.00 (m, 4H, H3, H4), 4.16 (Ψ-td, *J* 14.2(Ψ), 6.7, 6.7, 1H, H2), 4.55 (Ψ-td, *J* 39.1(Ψ), 5.7, 5.7, 1H, H5).

**cis-¹³C-NMR (75 MHz, CDCl₃:** δ = 28.14/27.84 (q, C8, C11), 30.39/29.71/29.64/28.57 (t, C3, C4), 47.52 (d, C5), 60.24/60.19 (d, C2), 82.01/81.91/81.62 (s, C7, C10), 118.38/118.09 (s, C12), 152.50/152.25 (s, C9), 170.53/170.30 (s, C6).

**cis-GC-MS:** m/z = 255 ([M]⁺, 1), 199 (5), 154 ([M]⁺-C₅H₉O₂, 100).

**cis-IR (FT-ATR):** ~v = 2976 (s), 2982 (m), 2880 (w), 2238 (w), 1738 (s), 1697 (s), 1473 (m), 1455 (m), 1384 (s), 1364 (s), 1286 (s), 1255 (s), 1215 (s), 1150 (s), 1116 (s), 1072 (s), 1028 (m), 986 (m), 950 (m), 935 (m), 905 (m), 878 (m), 842 (s), 769 (s).

**cis-HR-MS:** (ESI, C₁₅H₂₄N₂NaO₄): 319.163 ± 0.001 u (319.1634 u).

### (S)-Di-tert-butyl-5-formylpyrrolidin-1,2-dicarboxylat (111)

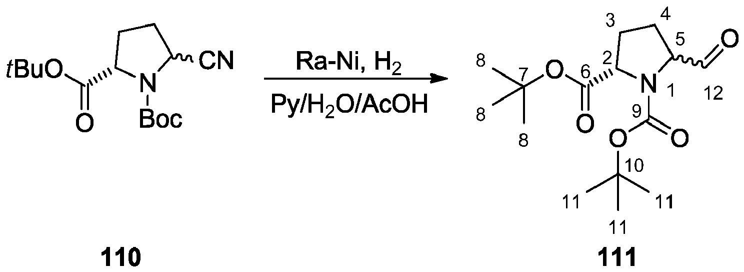

Zu einer Lösung von 17.905 g (60.42 mmol) des Cyanids **110** in 640 ml Py/AcOH/H₂O (2:1:1) wurden 90 g Raney-Ni/H₂O (50w% in Wasser, Acros) gegeben, und unter Wasserstoff 72 h bei 80 °C gerührt. Die Suspension wurde mit 300 ml Wasser versetzt und dreimal mit je 800 ml MTBE extrahiert. Die vereinigten organischen Phasen wurden zweimal mit Wasser gewaschen, über Magnesiumsulfat getrocknet und das Lösungsmittel bei vermindertem Druck entfernt. Der Rückstand wurde mit 1000 ml EtOAc/CyHex (1:1) über Kieselgel filtriert und das Rohprodukt nach Entfernen des Lösungsmittels durch Chromatographie an Kieselgel mit EtOAc/CyHex 1:4 als Laufmittel aufgereinigt. Es wurden 9.234 g (30.85 mmol, 51%) des Aldehyds **111** als gelbes Öl erhalten (d.r.: 1.7:1 *cis:trans*).

**M (C₁₅H₂₅NO₅):** 299.36 g mol⁻¹.

**DC:** R_{f} = 0.27 (EtOAc/CyHex 1:4).

**¹H-NMR (300 MHz, CDCl₃):** δ = 1.50-1.34 (m, 18H, H8, H11), 2.25-1.83 (m, 4H, H3, H4), 4.53-3.84 (m, 2H, H2, H5), 9.65-9.45 (m, 1H, H12).

**¹³C-NMR (75 MHz, CDCl₃:** δ = 29.94/29.20/29.00/28.27/26.29/26.17/25.24/24.56 (t, C3, C4), 28.14/27.90 (q, C8, C11), 60.52/60.38/60.29 (d, C5), 65.28 (d, C2), 81.63/81.54/81.45/81.35/81.23/81.00/80.77 (s, C7, C10), 154.12/153.93/153.55/153.15 (s, C9), 171.54/171.42/171.26 (s, C6), 200.99/200.11 (s,C12).

**GC-MS:** m/z = 299 ([M]⁺, 1), 270 ([M]⁺-CO, 5), 198 ([Mt-C₅H₉O₂, 2), 170 (27), 142 (8), 114 (100), 98 (41), 57 (75), 39 (25).

**IR (FT-ATR):** ~v = 2976 (s), 2926 (m), 2873 (w), 2806 (w), 2713 (w), 1730 (s), 1695 (s), 1476 (m), 1455 (m), 1383 (s), 1364 (s), 1290 (m), 1253 (s), 1220 (s), 1148 (s), 1123 (s), 1090 (s),1010 (m), 979 (m), 913 (m), 843 (m), 796 (w), 771 (m).

### (S)-Di-tert-butyl-5-vinylpyrrolidin-1,2-dicarboxylat (112)

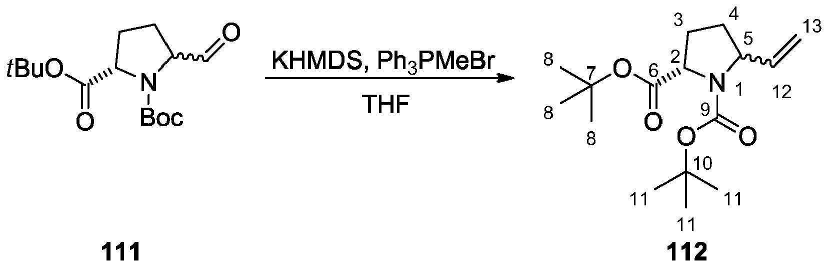

Zu einer Suspension von 22.037 g (61.69 mmol, 2.0 eq) Ph₃PMeBr in 220 ml THF wurden unter Argon bei RT 93.5 ml (61.69 mmol, 2.0 eq) einer Lösung von KHMDS in Toluol (15w%, ABCR) zugegeben und 1 h gerührt. Dann wurde bei -78 °C eine Lösung von 9.234 g (30.88 mmol, 1.0 eq) des Aldehyds **111** in 70 ml THF dazugetropft und 2.5 h bei RT gerührt. Das Reaktionsgemisch wurde mit 160 ml ges. Rochelle-Salz Lösung und 100 ml Wasser versetzt, dreimal mit je 260 ml MTBE extrahiert, die vereinigten organischen Phasen mit ges. NaCl-Lösung gewaschen und über Magnesiumsulfat getrocknet. Das Lösungsmittel wurde bei vermindetem Druck entfernt und nach säulenchromatographischer Aufreinigung an Kieselgel mit EtOAc/CyHex (1:9) als Laufmittel wurden 8.091 g (27.21 mmol, 88%) des Vinylprolins **112** als Isomerengemisch erhalten (d.r.: 1.7:1 *cis: trans*).

**M (C₁₆H₂₇NO₄):** 297.39 g mol⁻¹.

**DC:** R_{f} = 0.27 (EtOAc/CyHex 1:9).
**¹H-NMR (400 MHz, CDCl₃):** δ = 1.50-1.32 (m, 18H, H8, H11), 2.19-1.59 (m, 4H, H3, H4), 4.58-4.01 (m, 2H, H2, H5), 5.44-4.92 (m, 2H, H13), 5.95-5.60 (m, 1H, H12).

**¹³C-NMR (100 MHz, CDCl₃:** δ = 28.32/27.98 (q, C8, C11), 31.54/30.86/29.92/29.00/27.33 (t, C3, C4), 61.00/60.53/60.34/60.17/59.61/59.36 (d, C2, C5), 80.85/79.86/79.78/79.72 (s, C7, C10), 114.93/114.78/114.59/113.82/113.64 (t, C13), 139.21/138.97/138.63/138.50/138.06 (d, C12), 153.73/153.58 (s, C9), 172.09/172.03/171.97 (s, C6).

**GC-MS:** m/z = 297 ([M]⁺, 1), 196 ([M]-C₅H₉O₂, 168 (10), 140 (100), 114 (2), 96 (70), 79 (5), 57 (72), 39 (27).

**IR (FT-ATR):** ~v = 2975 (s), 2933 (m), 2880 (w), 1737 (s), 1697 (s), 1640 (w), 1477 (m), 1455 (m), 1385 (s), 1363 (s), 1323 (m), 1290 (m), 1255 (m), 1213 (m), 1150 (s), 1106 s), 1066 (w), 1023 (w), 988 (w), 957 (w), 912 (m), 873 (w), 856 (w), 843 (w), 770 (w).

**HR-MS:** (ESI, C₁₆H₂₇NNaO₄): 320.183 ± 0.001 u (320.1838 u).

### (2S,5S)-tert-Butyl-5-vinylpyrrolidin-2-carboxylat (113)

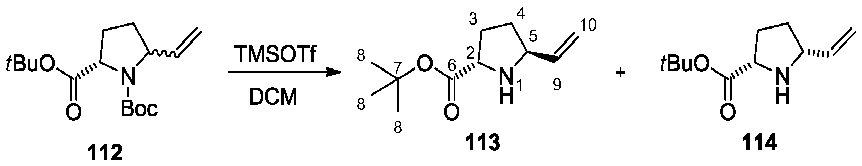

Zu einer Lösung von 4.041 g (13.59 mmol, 1.0 eq) des Vinylprolins **112** in 50 ml DCM wurden unter Argon bei 0 °C langsam 2.5 ml (13.82 mmol, 1.0 eq) TMSOTf gegeben, 5 min gerührt und die Reaktion durch Zugabe von 10 ml gesättigter NaHCO₃-Lsg beendet. Die wässrige Phase wurde dreimal mit je 16 ml DCM extrahiert, die vereinigten organischen Phasen über Magnesiumsulfat getrocknet und das Lösungsmittel bei vermindertem Druck entfernt. Nach Chromatographie an Kieselgel mit DCM/MeOH (25:1) als Laufmittel wurden 887 mg (4.50 mmol, 33%) des trans-Isomers **113** und 1.306 g (6.63 mmol, 49%) des cis-Isomers **114** erhalten werden.

**M (C₁₁H₁₉NO₂):** 197.27 g mol⁻¹.

**trans-DC:** R_{f} = 0.35 (DCM/MeOH 25:1).

**trans- [α]²⁰_{D}** : -28.9 (c = 0.715, CHCl₃).

**trans-¹H-NMR (300 MHz, CDCl₃):** δ = 1.42 (s, 9H, H8), 1.58-1.45 (m, 1H, H4'), 1.96-1.70 (m, 2H, H3, H4), 2.25-2.10 (m, 1H, H3'), 2.70 (s, 1H, NH), 3.77-3.65 (m, 2H, H2, H5), 4.97 (d, J 10.2, 1H, H10), 5.13 (dd, J 17.1, 0.9, 1H, H10'), 5.76 (ddd, J 17.1, 10.1, 7.0, 1H, H9).

**trans-¹³C-NMR (75 MHz, CDCl₃:** δ = 28.00 (q, C8), 29.60, 31.79 (t, C3, C4), 59.79, 60.89 (d, C2, C5), 81.01 (s, C7), 114.50 (t, C10), 140.89 (d, C9), 174.90 (s, C6).

**trans-GC-MS:** m/z = 197 ([M]+, 1), 96 ([M]+-C5H9O2, 100), 79 (12), 57 (22), 41 (32), 19 (10).
**trans-IR (FT-ATR):** ~v = 3346 (w), 3076 (w), 2974 (s), 2932 (m), 2872 (w), 1723 (s), 1641 (w), 1604 (w), 1591 (w), 1477 (m), 1456 (m) 1391 (s), 1366 (s), 1339 (m), 1226 (s), 1153 (s), 1029 (m), 991 (s), 916 (s), 846 (s), 808 (m), 754 (m) 721 (w), 692 (w), 677 (w).

**trans-HR-MS:** (ESI, C₁₁H₂₀NO₂) 198.149 ± 0.002 u (198.1494 u).

**cis-DC:** R_{f} = 0.27 (DCM/MeOH 25:1).

**cis- [α]²⁰D:** -25.1° (c = 1.475, CHCl₃).

**cis⁻¹H-NMR (400 MHz, CDCl₃):** δ = 1.44 (s, 9H, H8), 2.28-1.82 (m, 4H, H3, H4), 2.22 (s, 1H, NH), 3.59 (dd, *J* 14.3, 7.2 1H, H5), 3.67 (dd, *J* 8.7, 5.2 1H, H2), 5.03 (d, *J* 10.3, 1H, H10), 5.17 (d, *J* 17.1, 1H, H10') 5.85 (ddd, *J* 17.2, 10.2, 7.1, 1H, H9).

**cis⁻¹³C-NMR (100 MHz, CDCl₃:** δ = 28.02 (q, C8), 31.87, 30.37 (t, C3, C4), 60.70 (d, C2), 62.23 (d, C5), 81.13 (s, C7), 115.20 (t, C10), 139.97 (d, C9), 174.33 (s, C6).

**cis-GC-MS:** m/z =197 ([M]+, 1), 96 ([M]+-C5H9O2, 100), 79 (12), 57 (22), 41 (32), 19 (10).

**cis-IR (FT-ATR):** ~v = 3360 (w, br), 2977 (s), 2926 (m), 2873 (w), 1727 (s), 1636 (w), 1473 (w), 1453 (m), 1426 (w), 1390 (m), 1366 (s), 1283 (m), 1246 (s), 1225 (s), 1154 (s), 1100 (m), 1030 (w), 992 (m), 917 (m), 848 (s), 769 (w), 753 (w), 740 (w).

**cis-HR-MS:** (ESI, C₁₁H₂₀NO₂): 198.149 ± 0.001 u (198.1494 u).

### (2R)-tert-Butyl-2-((5S)-2-(tert- butoxycarbonyl)-5-vinylpyrrolidin-1-carbonyl)-2-vinylpyrrolidin-1-carboxylat (115/116)

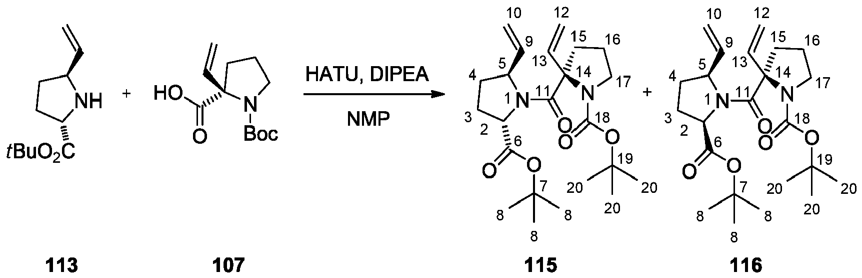

Eine Lösung von 743 mg (3.08 mmol, 1.0 eq) der Säure **107**, 1.288 g (3.39 mmol, 1.1 eq HATU und 1.02 ml (6.16 mmol, 2.0 eq) DIPEA in 15 ml NMP wurde 20 min bei RT gerührt. Dann wurden 792 mg (4.02 mmol, 1.3 eq des Amins **113** zugegeben und die Lösung 23 h auf 85 °C erhitzt. Nach Zugabe von 22 ml 10%iger wässriger Zitronensäurelsg. wurde dreimal mit je 40 ml MTBE extrahiert, die organischen Phasen mit je 120 ml ges. NaHCO₃-Lsg., ges. NaCl-Lsg. und Wasser gewaschen, über Magnesiumsulfat getrocknet und das Lösungsmittel bei vermindertem Druck entfernt. Durch Chromatographie an Kieselgel mit EtOAc/CyHex 1:3 als Laufmittel wurden 539 mg (1.28 mmol, 42%) eines Diastereomerengemisches (d.r.: 8:1 **115**:**116** der Dipeptide **115** und **116** als gelbliches Öl erhalten.

M (C₂₃H₃₆N₂O₅): 420.54 g mol⁻¹.

**DC:** R_{f} = 0.33 (EtOAc/CyHex 1:3).

**[α]²⁰_{D} :** -15.6 ° (c = 1.010, CHCl₃).

**¹H-NMR (600 MHz, CDCl₃):** δ = 1.45- 1.34 (m, 18H, H8, H20), 1.64-1.57 (m, 1H, H4'), 1.73-1.64 (m, 1H, H3'), 1.85-1.73 (m, 2H, H16), 2.09-2.01 (m, 1H, H3), 2.26-2.13 (m, 2H, H4, H15'), 2.34-2.26 (m, 1H, H15), 3.47-3.36 (m, 1H, H17'), 3.59-3.47 (m, 1H, H17), 4.37-4.26 (m, 0.5H, H2), 4.71 (s, 0.5H, H5), 4.80-4.74 (m, 0.5H, H2), 4.82 (s, 0.5H, H5), 5.10-4.95 (m, 3H, H10', H12), 5.20-5.10 (m, 1H, H10), 5.83-5.63 (m, 1H, H9), 6.69-6.55 (m, 1H, H13).

**¹³C-NMR (150 MHz, CDCl₃:** δ = 22.41, 21.63 (t, C16), 25.08, 24.85 (t, C3), 28.27, 27.86 (q, C8, C20), 31.30, 30.69 (t, C4), 36.78, 35.93 (t, C15), 48.31, 48.02 (t, C17), 59.97, 59.69 (d, C5), 61.80, 61.60 (d, C2), 70.71, 70.15 (s, C14), 80.83, 80.66 (s, C7, C19), 112.15 (t, C12), 114.62 (t, C10), 139.75, 138.56 (d, C9), 140.08, 140.02 (d, C13), 154.27, 154.04 (s, C18), 171.40 (s, C11), 171.08 (s, C6).

**IR (FT-ATR):** ~v = 3073 (w), 2973 (m), 2926 (w), 2880 (1), 1735 (s), 1693 (s), 1634 (s), 1477 (w), 1455 (w), 1380 (s), 1363 (s), 1300 (w), 1255 (m), 1217 (m), 1149 (s), 1075 (w), 992 (w), 917 (w), 850 (w), 769 (w), 663 (w).

**HR-MS:** (ESI, C₂₃H₃₆N₂NaO₅): 443.253 ± 0.002 u (443.2522 u).

### (2'R,3S,8aS)-Di-tert-butyl-5-oxo-2,3,5,8a-tetrahydro-1H-spiro[indolizin-6,2' pyrrolidin]-1',3-dicarboxylat (117)

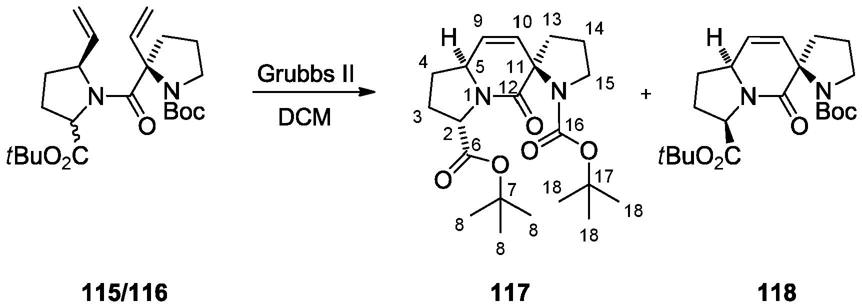

Eine Lösung von 135 mg (0.32 mmol, 1.0 eq) der Dipeptide **115** und **116** und 82 mg (0.10 mmol, 0.3 eq) Grubbs II in 6.5 ml DCM wurde 7 h bei 55 °C in der Mikrowelle (300 W) erhitzt. Nach Zugabe von 0.5 ml DMSO wurde über Nacht bei RT gerührt und das Lösungsmittel dann bis 100 mbar bei vermindertem Druck entfernt. Durch Chromatographie an Kieselgel mit EtOAc/CyHex 1:1 1 als Laufmittel wurden 65 mg (0.17 mmol, 53%) des Produktes **117** und 17 mg (0.04 mmol, 13%) des Diastereomers **118** als grauer Feststoff erhalten. Es konnten außerdem 30 mg (0.07 mmol, 22%) des Eduktes **115/116** reisoliert werden.

**M (C₂₁H₃₂N₂O₅):** 392.49 g mol⁻¹.

**DC:** R_{f} = 0.30 (EtOAc/CyHex 1:1).

**[α]²⁰_{D} :** -138.7 ° (c = 0.265, CHCl₃).

**Schmelzpunkt**: 128.0 °C.

**¹H-NMR (600 MHz, CDCl₃, Rotamerengemisch; Rot1:Rot2: 1.5:1): Rotamer 1**: δ = 1.30 (s, 9H, H18), 1.44 (s, 9H, H8), 1.56-1.48 (m, 1H, H4'), 1.91-1.75 (m, 3H, H3', H13', H14'), 2.04-1.96 (m, 1H, H14), 2.14-2.10 (m, 1H, H4), 2.27-2.20 (m, 1H, H13), 2.45-2.36 (m, 1H, H3), 3.50-3.41 (m, 1H, H15'), 3.78-3.72 (m, 1H, H15), 4.22-4.16 (m, 1H, H5), 4.46 (t, J 8.8, 1H, H2), 5.72 (dd, J 9.9, 1.4, 1H, H10), 5.79 (dd, J9.8, 2.8, 1H, H9); **Rotamer 2**: δ = 1.40 (s, 9H, H18), 1.43 (s, 9H, H8), 1.75-1.69 (m, 1H, H4'), 1.91-1.75 (m, 3H, H3', H13', H14'), 2.04-1.96 (m, 1H, H14), 2.10-2.05 (m, 1H, H4), 2.27-2.20 (m, 1H, H13), 2.45-2.36 (m, 1H, H3), 3.50-3.41 (m, 1H, H15'), 3.71-3.65 (m, 1H, H15), 4.22-4.16 (m, 1H, H5), 4.56 (t, *J* 8.6, 1H, H2), 5.80 (dd, *J* 9.6, 1.4, 1H, H10), 5.88 (dd, *J* 9.8, 2.7, 1H, H9).

**¹³C-NMR (150 MHz, CDCl₃, Rotamerengemisch):** δ = 22.80, 22.49 (t, C14), 28.21, 28.14 (t, C3), 28.52, 28.32, 27.99 (q, C8, C18), 31.30, 31.14 (t, C4), 39.43, 38.15 (t, C13), 48.26, 47.98 (t, C15), 57.84, 57.62 (d, C2), 58.64, 58.52 (d, C5), 64.28, 64.00 (s, C11), 79.63, 79.39 (s, C17), 81.33, 81.08 (s, C7), 122.46, 121.41 (d, C10), 134.03, 133.37 (d, C9), 154.21, 154.07 (s, C16), 168.05, 167.93 (s, C12), 171.54, 171.25 (s, C6).

**IR (FT-ATR):** ~v = 2972 (s), 2929 (m), 2873 (m), 1735 (s), 1696 (s), 1659 (s), 1477 (m), 1432 (s), 1383 (s), 1364 (s), 1293 (m), 1255 (s), 1211 (s), 1149 (s), 1078 (w), 1034 (w), 1004 (m), 959 (w), 928 (w), 884 (w), 870 (w), 843 (w), 803 (w), 786 (w), 770 (w), 734 (w), 702 (w).

### Kristalldaten 117

| | | |
|---|---|---|
| Identification code | cr297-2 | |
| Empirical formula | C21 H32 N2 05 | |
| Formula weight | 392.49 | |
| Temperature | 100(2) K | |
| Wavelength | 0.71073 A | |
| Crystal system, space group | Monoclinic, P21 | |
| Unit cell dimensions | a = 14.4763(17) A | alpha = 90 deg. |
| | b = 6.8044(4) A | beta = 102.213(4) deg. |
| | c = 22.803(3) A | gamma = 90 deg. |
| Volume | 2195.4(4) A^3 | |
| Z, Calculated density | 4, 1.187 Mg/m^3 | |
| Absorption coefficient | 0.084 mm^-1 | |
| F(000) | 848 | |
| Crystal size | .3 x.075 x.05 mm | |
| Theta range for data collection | 2.07 to 27.00 deg. | |
| Limiting indices | -18<=h<=15 -8<=k<=5 -29<=1<=21 | |
| Reflections collected / unique | 9852 / 5126 [R(int) = 0.0693] | |
| Reflection observed [1>2sigma(I)] | 2646 | |
| Completeness to theta = 27.00 | 98.2 % | |
| Absorption correction | None | |
| Refinement method | Full-matrix least-squares on F^2 | |
| Data / restraints / parameters | 5126 / 1 / 517 | |
| Goodness-of-fit on F^2 | 0.828 | |
| Final R indices [I>2sigma(I)] | R1 = 0.0491, wR2 = 0.0705 | |
| R indices (all data) | R1 = 0.1398, wR2 = 0.0885 | |
| Absolute structure parameter | -0.3(12) | |
| Largest diff. peak and hole | 0.193 and -0.202 e.A^-3 | |

### Kristalldaten 118

| | | |
|---|---|---|
| Identification code | z2 | |
| Empirical formula | C21 H32 N2 05 | |
| Formula weight | 392.49 | |
| Temperature | 100(2) K | |
| Wavelength | 0.71073 A | |
| Crystal system, space group | Monoclinic, P21 | |
| Unit cell dimensions | a = 10.5025(9) A | alpha = 90 deg. |
| | b = 8.0873(10) A | beta = 94.484(6) deg. |
| | c = 12.6674(13) A | gamma = 90 deg. |
| Volume | 1072.6(2) A^3 | |
| Z, Calculated density | 2, 1.215 Mg/m^3 | |
| Absorption coefficient | 0.086 mm^-1 | |
| F(000) | | 424 |
| Crystal size | .2 x .1 x .05 mm | |
| Theta range for data collection | 1.94 to 26.99 deg. | |
| Limiting indices | #NAME? | |
| Reflections collected / unique | 5802 / 2477 [R(int) = 0.0346] | |
| Reflection observed [1>2sigma(I)] | | 1970 |
| Completeness to theta = 26.99 | 98.6 % | |
| Absorption correction | None | |
| Refinement method | Full-matrix least-squares on F^2 | |
| Data / restraints / parameters | 2477 / 1 / 259 | |
| Goodness-of-fit on F^2 | | 1.047 |
| Final R indices [1>2sigma(I)] | R1 = 0.0485, wR2 = 0.1243 | |
| R indices (all data) | R1 = 0.0660, wR2 = 0.1334 | |
| Absolute structure parameter | -0.9(15) | |
| Largest diff. peak and hole | 0.649 and -0.294 e.A^-3 | |

### (2'R,3S,8aS)-1'-(((9H-Fluoren-9-yl)methoxy)carbonyl)-5-oxo-2,3,5,8a tetrahydro-1H spiro[indolizin-6,2'-pyrrolidin]-3-carbonsäure (85)

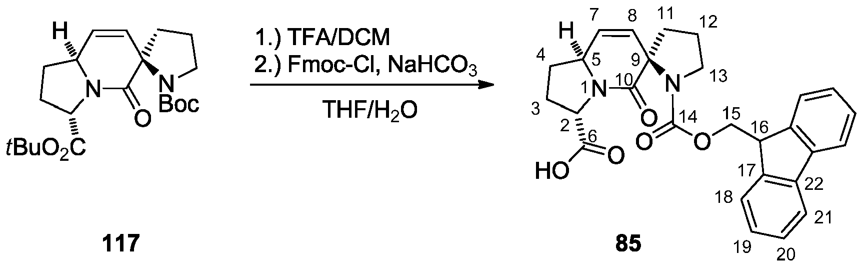

Zu einer Lösung von 49 mg (0.13 mmol, 1.0 eq) des Dipeptids **117** in 2 ml DCM wurden bei 0 °C 2 ml TFA gegeben und anschließend 1 h bei RT gerührt. Das Lösungsmittel wurde bei vermindertem Druck entfernt, der Rückstand in 3 ml halbgesättigter NaHCO₃-Lsg. aufgenommen und der pH-Wert kontrolliert (pH ≥ 8). Dann wurde bei 0 °C eine Lösung von 51 mg (0.20 mmol, 1.5 eq) Fmoc-Cl in 1 ml THF zugegeben und über Nacht bei RT gerührt. Nach Zugabe von 5 ml DCM wurde die Lösung bei 0 °C mit 1 N HCl-Lsg. auf pH = 1 gebracht, die Phasen getrennt und die wässrige Phase zweimal mit DCM extrahiert. Die vereinigten organischen Phasen wurden über Magnesiumsulfat getrocknet, das Lösungsmittel bei vermindertem Druck entfernt und durch Chromatographie an Kieselgel mit DCM/MeOH 15:1 als Laufmittel wurden 53 mg (0.11 mmol, 85%) des Fmoc-geschützten Produktes **85** erhalten.

**M (C₂₇H₂₆N₂O₅):** 458.51 g mol⁻¹.

**DC:** R_{f} = 0.20 (DCM/MeOH 15:1).

### Schmelzpunkt:

**¹H-NMR (600 MHz, MeOD, Rotamerengemisch; Rot1:Rot2 1:1):** δ = **Rotamer 1:** δ = 1.66-1.55 (m, 1H, H4'), 2.03-1.77 (m, 4H, H3', H11', H12), 2.16-2.08 (m, 1H, H4), 2.34-2.28 (m, 1H, H11), 2.42-2.35 (m, 1H, H3), 3.54-3.45 (m, 1H, H13'), 3.71-3.61 (m, 1H, H13), 4.33-4.16 (m, 3H, H5, H15', H16), 4.41 (t, *J* 8.7, 1H, H2), 4.54 (dd, *J* 10.9, 6.0, 1H, H15), 5.69 (dd, *J* 10.0, 2.4, 1H, H7), 5.87 (dd, *J* 10.0, 1.1, 1H, H8), 7.32-7.26 (m, 2H, H19), 7.40-7.34 (m, 2H, H20), 7.63-7.50 (m, 2H, H18), 7.79-7.72 (m, 2H, H21); **Rotamer 2:** 1.24-1.15 (m, 1H, H4'), 2.03-1.77 (m, 5H, H3', H4, H11', H12), 2.28-2.23 (m, 1H, H3), 2.34-2.29 (m, 1H, H11), 3.54-3.45 (m, 1H, H13'), 3.71-3.61 (m, 1H, H13), 4.03 (t, *J* 5.6, 1H, H16), 4.17-4.11 (m, 1H, H5), 4.33-4.17 (m, 3H, H2, H15), 5.48 (dd, *J* 9.8, 2.6, 1H, H7), 5.58 (d, *J* 9.9, 1H, H8), 7.32-7.26 (m, 2H, H19), 7.40-7.34 (m, 2H, H20), 7.63-7.50 (m, 2H, H18), 7.79-7.72 (m, 2H, H21).

**¹³C-NMR (150 MHz, MeOD, Rotamerengemisch):** δ = 24.19/23.25 (t, C12), 29.53/29.45 (t, C3), 32.09/32.05 (t, C4), 40.35/39.46 (t, C11), 48.34 (d, C16), 49.90/49.43 (t, C13), 60.64/60.13 (d, C5), 61.52/61.31 (d, C2), 65.75/65.72 (s, C9), 68.42/67.85 (t, C15), 121.00/120.93 (d, C21), 124.58/124.35 (d, C8), 126.13/126.09/125.97/125.77 (d, C18), 128.12/128.04/127.99 (d, C19), 128.80/128.73 (d, C20), 132.77/131.64 (d, C7), 142.57/142.51 (s, C22), 145.51/145.36, 145.05/145.01 (s, C17), 156.69/155.97 (s, C14), 170.20/170.10 (s, C10), 180.14/179.96 (s, C6).

### Abkürzungen

- abs.: absolut
- Äq.: Äquivalent(e)
- Ar: Aryl
- ATR: Attenuated Total Internal Reflectance
- 9-BBN: 9-Borabicyclononan
- ber.: berechnet
- Bn: Benzyl
- Boc: *tert*-Butoxycarbonyl
- Boc₂O: Di-*tert*-butyldicarbonat (Boc-Anhydrid)
- CH: Cyclohexan
- Cy: Cyclohexyl
- DC: Dünnschichtchromatogramm
- DCE: Dichlorethan (1,2-)
- DCM: Dichlormethan
- DIBAL-H: Diisobutylaluminiumhydrid
- DIC: Diisopropylcarbodiimid
- DIP: Direkteinlass (Massenspektrometrie)
- DIPEA: Diisopropylethylamin
- DMAP: 4-*N*,*N*-Dimethylaminopyridin
- DMF: *N*,*N*-Dimethylformamid
- DMP: 2,2-Dimethoxypropan
- DMS: Dimethylsulfid
- DMSO: Dimethylsulfoxid
- dr: Diastereomerenverhältnis (diastereomeric ratio)
- EE: Ethylacetat
- ee: Enantiomerenüberschuss (enantiomeric excess)
- EI: Elektronenstoßionisation
- ESI: Elektronensprayionisation
- Et: Ethyl
- Et₂O: Diethylether
- EtOH: Ethanol
- EVH1: Ena-VASP-Homologie-1-Domäne
- FGI: Funktionelle-Gruppen-Chemie (function group inversion)
- Fmoc: Fluorenyl-9-methoxycarbonyl
- FMP: Forschungsinstitut für Molekulare Pharmakologie (Berlin-Buch)
- GC-MS: Gaschromatographie mit angeschlossener Massenspektrometrie
- gef.: gefunden
- ges.: gesättigt
- HPLC: High Performance Liquid Chromatography
- HRMS: hochaufgelöste Massenspektrometrie
- IR: Infrarotspektroskopie
- konz.: konzentriert
- LiHMDS: Lithium-hexamethyldisilazid
- M: molare Masse
- MCPBA: *meta*-Chlorperbenzoesäure
- Me: Methyl
- MeOH: Methanol
- Ms: Mesyl (Methansulfonyl)
- MS: Massenspektrometrie
- MTBE: Methyl-*tert*-butylether
- NaHMDS: Natrium-hexamethyldisilazid
- NME: *N*-Methylephedrin
- NMR: Magnetische Kernresonanzspektroskopie
- NOE: Nuclear Overhauser Effect
- *o*: *ortho*
- Ph: Phenyl
- PPII: Polyprolin-Helix Typ II
- POM-C1: Chlormethylpivaloat
- PPTS: Pyridinium-para-toluolsulfonat
- RCM: Ringschlussmetathese
- R_{f}: Retentionsfaktor
- [Ru]II: Grubbs-II-Katalysator **81**
- [Ru]_{gr}: modifizierter (grüner) Grubbs-Hoveyda-Katalysator nach Blechert **82**
- s: sekundär
- Smp.: Schmelzpunkt
- Su: Succinimid
- TBAF: Tetrabutylammoniumfluorid
- TBS: *tert*-Butyldimethylsilyl
- *t*-Bu: *tert*-Butyl
- Tf: Trifluormethansulfonyl
- TFA: Trifluoressigsäure
- THF: Tetrahydrofuran
- TMEDA: Tetramethylethylendiamin
- TMS: Trimethylsilyl
- TMSOTf: Trimethylsilyl-trifluormethansulfonat
- TPS: *tert*-Butyldiphenylsilyl
- Ts: Tosyl (*para*-Toluolsulfonyl)
- TsOH: para-Toluolsulfonsäure
- Z: Benzyloxycarbonyl- (auch Cbz)

Ein-Buchstaben-Code und Drei-Buchstaben-Code natürlicher proteinogener Aminosäuren:

| | | | | | |
|---|---|---|---|---|---|
| A | Ala | Alanin | M | Met | Methionin |
| C | Cys | Cystein | N | Asn | Asparagin |
| D | Asp | Asparaginsäure | p | Pro | Prolin |
| E | Glu | Glutaminsäure | Q | Gln | Glutamin |
| F | Phe | Phenylalanin | R | Arg | Arginin |
| G | Gly | Glycin | S | Ser | Serin |
| H | His | Histidin | T | Thr | Threonin |
| I | Ile | Isoleucin | V | Val | Valin |
| K | Lys | Lysin | W | Trp | Tryptophan |
| L | Leu | Leucin | Y | Tyr | Tyrosin |

## Patentansprüche

1. Verbindungen gemäß der allgemeinen Formel 1 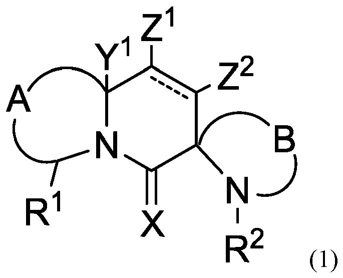 mit einem gesättigten oder ungesättigten zentralen sechsgliedrigen Ring, wobei
X = O und/oder S sind;
A, B = Ringbrücken sind;
Y¹= H, Alkyl, Fluoralkyl, Aryl und/oder Heteroaryl sind;
Z¹, Z² = H, Carbonyl, OH, O-Alkyl, O-Acyl, N-R¹R² mit R¹ bzw. R² = H, Alkyl, Acyl, Sulfonyl, Alkyl, Acyl, Fluoralkyl, Aryl und/oder Heteroaryl sind;
R¹ = Alkyl, Acyl, Alkoxycarbonyl, Aryloxycarbonyl und/oder Aminocarbonyl (CONH₂, CONHR, CONH-Peptidyl, (mit R)) ist;
R² = H, Alkyl, Aryl, Alkylcarbonyl, Arylcarbonyl, Alkoxycarbonyl, Aminocarbonyl, Aryloxycarbonyl, Alkylsulfonyl, Arylsulfonyl, Aminoacyl und/oder Peptidyl sind.

2. Verbindung nach Anspruch 1
**dadurch gekennzeichnet, dass**
A und/oder B 5 und/oder 6 Ringatome sind, wobei die durch A und B repräsentierten Ringglieder ausgewählt sind aus der Gruppe umfassend C-, O-, S- und/oder N-Atome.

3. Verbindung nach Anspruch 1 oder 2 gemäß der allgemeinen Formel 2 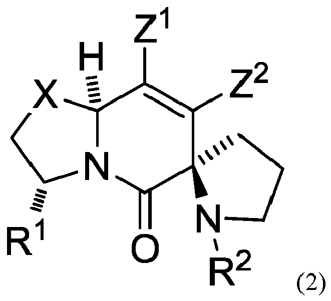
mit Z¹, Z² wie für Struktur 1 angegeben und der im Formelbild 2 gezeigten Konfiguration,
mit R¹, R² = Alkyl, Acyl, Hetaryl, Sulfonyl,
mit X = -CH₂-, -O-, -S- und -NH-R.

4. Verbindung nach Anspruch 1 bis 3,
**dadurch gekennzeichnet, dass**
der bevorzugten Ausführungsform gemäß der in Formel 3 gezeigten Strukturen entsprechen 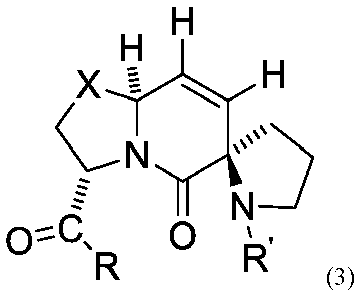 mit X = --CH₂-, -O-, -S-,
mit R = NH-R", -O-R", mit R" = Peptidyl, substituierte Alkyle, Hetaryl,
mit R' = Acyl, Peptidyl, Sulfonyl.

5. Verbindungen gemäß einem der Ansprüche 1 bis 4 als pharmazeutische Wirkstoffe.

6. Pharmazeutisches Mittel umfassend eine Verbindung gemäß einem der Ansprüche 1 bis 4, ggf. zusammen mit einem pharmazeutisch verträglichen Träger.

7. Pharmazeutisches Mittel nach dem vorhergehenden Anspruch,
**dadurch gekennzeichnet, dass**
die pharmazeutischen Träger ausgewählt sind aus der Gruppe umfassend Füllmittel, Streckmittel, Bindemittel, Feuchthaltemittel, Lösungsverzögerer, Sprengmittel, Resorptionsbeschleuniger, Netzmittel, Absorptionsmittel und/oder Gleitmittel.

8. Verwendung der Verbindungen gemäß einem der Ansprüche 1 bis 7 als Ligand für eine Domäne ausgewählt aus der Gruppe umfassend Src-Homologie-3-Domänen, WW-Domänen, Ena-VASP-Homologie-1-Domänen, GYF-Domänen, UEV-Domänen und/oder Profilin.

9. Verwendung nach dem vorhergehenden Anspruch, wobei die Verbindungen gemäß der Ansprüche 1 bis 7 als Poly-Prolin-Mimetika eingesetzt werden.

10. Verwendung der pharmazeutischen Mittel nach Anspruch 6 oder 7 zur Behandlung von Krankheiten, die mit der Modifikation von intrazellulären Signaltransduktionsvorgängen assoziiert sind, die durch Poly-Prolin-Helix-Strukturen vermittelt werden, ausgewählt aus der Gruppe umfassend bakterielle Infektionskrankheiten, neurodegenerative Erkrankungen und/oder Tumorerkrankungen.

11. Verwendung nach dem vorhergehenden Anspruch,
**dadurch gekennzeichnet, dass**
die bakteriellen Erkrankungen durch Bakterien ausgelöst werden, ausgewählt aus der Gruppe umfassend Legionellen, Streptokokken, Staphylokokken, Klebsiellen, Haemophilis influenzae, Rickettsien (Fleckfieber), Mykobakterien, Mykoplasmen, Ureaplasmen, Neisserien (Meningitis, Waterhouse-Friedrichsen-Syndrom, Gonorrhoe), Pseudomonaden, Bordetellen (Pertussis), Corynobakterien (Diphtherie), Chlamydien, Campylobacter (Durchfall), Escherichia coli, Proteus, Salmonellen, Shigellen, Yersinien, Vibrionen, Enterokokken, Clostridien, Borrelien, Treponema pallidum, Brucellen, Francisellen und/oder Leptospira, insbesondere Listerien.

12. Verwendung nach dem vorhergehenden Anspruch,
**dadurch gekennzeichnet, dass**
die Listerien ausgewählt sind aus der Gruppe umfassend L. monocytogenes Sv1/2a, L. monocytogenes Sv4b F2365, L. monocytogenes Sv4b H7858, 178 contigs, L. monocytogenes Sv1/2a F6854, 133 contigs, L. monocytogenes Sv4b, L. monocytogenes Sv4a, L. innocua Sv6a, L. welshimeri Sv6b, L. seeligeri Sv1/2b und/oder L. ivanovii Sv5.

13. Verwendung nach einem der vorhergehenden Ansprüche,
**dadurch gekennzeichnet, dass**
die neurodegenerative Erkrankung ausgewählt ist aus der Gruppe umfassend Morbus Alzheimer, Morbus Parkinson, Morbus Huntington und/oder amyotrophische Lateralsklerose (ALS).

14. Verwendung nach einem der vorhergehenden Ansprüche,
**dadurch gekennzeichnet, dass**
die Tumorerkrankung ein Karzinom, ein Sarkom, ein neuroendokriner Tumor, ein hämoonkologischer Tumor, ein dysontogenetischer Tumor und/oder ein Mischtumor ist.

15. Verwendung nach einem der vorhergehenden Ansprüche,
**dadurch gekennzeichnet, dass**
die Tumorerkrankung ausgewählt ist aus der Gruppe umfassend Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehirnmetastasen, Lungen-metastasen, Lebermetastasen, Knochenmetastasen, pleurale und perikardiale Metastasen und maligne Aszites.
